# EUROPEAN PATENT APPLICATION

(11) **EP 2 759 295 A1**
(43) Date of publication of application: **30.07.2014**
(21) Application number: 13153142.8
(22) Date of filing: 29.01.2013
(51) Int. Cl.: A61K 31/05, A61K 31/352

(54) **Foam cell specific Liver X Receptor (LXR) alpha agonist, SIRT1 inhibitors as well as p300 inhibitors as pharmaceutically active agents**

(71) Applicant: Max-Planck-Gesellschaft zur Förderung der Wissenschaften e.V., 80539 München (DE)
(72) Inventor: Sauer, Sascha, 10711 Berlin (DE); Holzhauser, Susanne, 10627 Berlin (DE); Feldmann, Radmila, 40211 Düsseldorf (DE); Geikowski, Anne, 12249 Berlin (DE)
(74) Representative: Arth, Hans-Lothar

(57) **Abstract**

The present invention relates to compound (I) and its stereoisomeric forms, prodrugs, solvates, hydrates and/or pharmaceutically acceptable salts of these compounds as well as pharmaceutical compositions containing at least one of these compounds together with pharmaceutically acceptable other LXRα agonist, carrier, excipient and/or diluents. Said compound (I), an LXRα agonist has been identified as useful for the prophylaxis and treatment of atherosclerosis and cardiovascular diseases.

Further, the present invention refers to compound (II) and its stereoisomeric forms, prodrugs, solvates, hydrates and/or pharmaceutically acceptable salts of these compounds as well as pharmaceutical compositions containing at least one of these compounds together with pharmaceutically acceptable carrier, excipient and/or diluents. Said compound (II), a SIRT1 inhibitor has been identified as useful for the prophylaxis and treatment of neurodegenerative diseases or cancer.

Furthermore, the present invention discloses compound (III) and its stereoisomeric forms, prodrugs, solvates, hydrates and/or pharmaceutically acceptable salts of these compounds as well as pharmaceutical compositions containing at least one of these compounds together with pharmaceutically acceptable carrier, excipient and/or diluents. Said compound (III), a p300 inhibitor has been identified as useful for the prophylaxis and treatment of cancer, metabolic, inflammatory, neurodegenerative or cardiovascular diseases.

## Description

### Specification

The present invention relates to compound (I) and its stereoisomeric forms, prodrugs, solvates, hydrates and/or pharmaceutically acceptable salts of these compounds as well as pharmaceutical compositions containing at least one of these compounds together with pharmaceutically acceptable other LXRα agonist, carrier, excipient and/or diluents. Said compound (I), an LXRα agonist has been identified as useful for the prophylaxis and treatment of atherosclerosis and cardiovascular diseases.

Further, the present invention refers to compound (II) and its stereoisomeric forms, prodrugs, solvates, hydrates and/or pharmaceutically acceptable salts of these compounds as well as pharmaceutical compositions containing at least one of these compounds together with pharmaceutically acceptable carrier, excipient and/or diluents. Said compound (II), a SIRT1 inhibitor has been identified as useful for the prophylaxis and treatment of neurodegenerative diseases or cancer.

Furthermore, the present invention discloses compound (III) and its stereoisomeric forms, prodrugs, solvates, hydrates and/or pharmaceutically acceptable salts of these compounds as well as pharmaceutical compositions containing at least one of these compounds together with pharmaceutically acceptable carrier, excipient and/or diluents. Said compound (III), a p300 inhibitor has been identified as useful for the prophylaxis and treatment of cancer, metabolic, inflammatory, neurodegenerative or cardiovascular diseases.

### Background of the invention

### 1. LXRα agonists

Atherosclerosis and cardiovascular diseases have become enormous health problems during the last decades. Macrophages play an important role in the development and progression of atherosclerosis [Moore et al. Cell (2011) 145, 341-355]. A key process of atherosclerosis involves the uncontrolled uptake of oxidized low density lipoproteins (oxLDL) in macrophages, which agglomerate at the subendothelial space of blood vessel walls [ Tabas I. Nature Medicine (2011), 17, 791-793]. When macrophages fail to restore cellular cholesterol homeostasis via regulation of reverse cholesterol transport (RCT) they form diseased foam cells [Cuchel M. et al. Circulation (2006), 113, 2548-2555.]. The nuclear receptors (Liver X Receptor alpha) and LXRβ act as cholesterol sensors and respond to elevated oxysterol levels by inducing target genes with impact on cholesterol metabolism and atherosclerosis [Chinetti-Gbaguidi G. et al. Biochim. Biophys. Acta (2009), 1791, 486-493]. Many approaches have been pursued to find LXR ligands with exclusively beneficial properties [ Joseph S.B. et al. Proc. Natl. Acad. Sci. (2002), 99, 7604-7609; Zanotti I. et al. J. Lipid Res. (2008), 49, 954-960].However, LXRs are also implicated in other counteracting physiological processes, such as triglyceride synthesis [Schultz J.R. et al. Genes. Dev. (2000), 14, 2831-2838]. A current research aims to develop selective LXR modulators as biochemical tools for mechanistic studies on LXR biology and as pharmaceutically exploitable compounds without exhibiting mentioned adverse side-effects [Jakobsson T. et al. Trends Pharmacol Sci (2012), 33, 394-404].

Agonists for the ubiquitously expressed subtype LXRβ have in general low efficiency to counteract atherogenic processes. Recent results suggest that rather the LXRα subtype plays a crucial role for regulating anti-atherogenic gene expression profiles [Bischoff E.D. et al. J. Lipid Res.(2010), 51, 900-906]. In human macrophages LXRα is controlled by a feed-forward autocatalytic loop resulting in increased levels by LXRα ligand-treatment [Laffitte B.A. et al. Mol. Cell Biol. (2001), 21, 7558-7568].

In the present invention an LXRα ligand is disclosed that specifically activates target genes in diseased human foam cells but not in macrophages. This activation resulted in significant reduction of total and esterified cholesterol with similar potency as for the synthetic LXRα/β ligand T0901317 (T09) - without the observed counteracting increases of triglyceride levels known from previously published LXRα/β ligands.

It is the objective of the present invention to provide compounds (I) and/or pharmaceutically acceptable salts thereof which can be used as pharmaceutically active agents, especially for the treatment of atherosclerosis and cardiovascular diseases as well as compositions comprising at least one of those compounds (I) and/or pharmaceutically acceptable salts thereof as pharmaceutically active ingredients.

### 2. SIRT1 inhibitors

In humans, the sirtuin family of deacetylase enzymes comprises seven proteins (SIRT1-7) that are dependent on NAD+ for their activity. Three protein subtypes are located in the nucleus, three in the mitochondria and only one is predominantly cytoplasmic. Caloric restriction and oxidative stress generally up-regulate their expression. SIRT1, the orthologue of yeast Sir2, is the mammalian sirtuin that has been most extensively studied to date. Among other targets, SIRT1 down-regulates the activity of the nuclear transcription factor p53, being this related with an increase in cell survival associated to stress resistance. Because sirtuin modulation could have beneficial effects on several human diseases, there is a growing interest in the discovery and development of small molecules that modify its activity.

The present invention relates to SIRT1 inhibitors. Inhibition of SIRT1 is useful for the treatment of some kinds of cancer. A US patent application US2007/0197459 A1 disclosed the induction of apoptosis in tumour cells by inhibition of SIRT1 expression. SIRT1 interacts with p53 via the p53 core and carboxy-terminus. It appears to act as a p53 deacetylase, as overexpression of SIRT1 results in reduced acetylation of p53. SIRT1 negatively regulates p53 function via deacetylation of p53, so that inhibition of SIRT1 function sensitizes cells to p53-dependent apoptosis in response to cellular stress. Recently, it was shown that a small molecule suppressing SIRT1 activity and inducing the acetylation level and activity of p53, consequently and effectively represses the growth of xenograft tumours derived from human lung and colon WT p53-containing cancer cells [Zhang Q. et al. EMBO Mol Med. (2012), 4(4), 298-312].

SIRT1 inhibition increases p53 acetylation and transcriptional activity in CML progenitors, and the inhibitory effects of SIRT1 targeting on CML cells depended on p53 expression and acetylation. Activation of p53 via SIRT1 inhibition represents a potential approach to target CML LSC (Leukemia Stem Cells) [Li L. et al. Cancer Cell (2012), 21 (2), 266-281].

Neurodegeneration is the umbrella term for the progressive loss of structure or function of neurons, including death of neurons. Many neurodegenerative diseases including Parkinson's, Alzheimer's, and Huntington's occur as a result of neurodegenerative processes. As research progresses, many similarities appear which relate these diseases to one another on a sub-cellular level. Discovering these similarities offers hope for therapeutic advances that could ameliorate many diseases simultaneously. There are many parallels between different neurodegenerative disorders including atypical protein assemblies as well as induced cell death. Neurodegeneration can be found in many different levels of neuronal circuitry ranging from molecular to systemic.

For the treatment of Huntington's Disease, the SIRT1 (sirtuin-1) inhibitor EX-527 (also known as SEN0014196) is currently in a Phase 1a combined single and multiple ascending dose study in the European Union.

It is the second objective of the present invention to provide compounds (II) and/or pharmaceutically acceptable salts thereof which can be used as pharmaceutically active agents, especially for the treatment of neurodegenerative diseases or cancer as well as compositions comprising at least one of those compounds (II) and/or pharmaceutically acceptable salts thereof as pharmaceutically active ingredients.

### 3. p300 inhibitors

Histone acetyltransferases (HATs) modulate gene expression by catalyzing targeted acetylation of the ε-amino group of lysine residues on histones and nonhistone proteins. HATs can be classified into several families based on the number of highly conserved structural motifs. p300/CBP family is one of them. p300 is a ubiquitously expressed global transcriptional coactivator that has critical roles in a wide variety of cellular phenomena including cell cycle control, differentiation, and apoptosis. The transcriptional coactivator function of this protein is partially facilitated by its intrinsic HAT activity. Significantly, p300 also acetylates several nonhistone proteins with functional consequences.

p300 shows opposing functions than SIRT1, as it shares a number of target proteins and acetylates proteins which are deacetylated by SIRT1. SIRT1 activation has been related to many health beneficial effects, but direct activation of SIRT1 by small molecules has been doubted in the past due to assay artifacts. Therefore, inhibition of the opposing enzyme, p300, may display similar effects as SIRT1 activation. Further research will be needed to deeper understand the SIRT1-counteracting roles of p300.

Therapeutic indication for SIRT1 activation or p300 inhibition includes cancer, metabolic diseases, cardiovascular diseases, neurodegenerative diseases and inflammation. Overexpression or activation of SIRT1 has been shown to modulate mitochondrial biogenesis, metabolic rate, insulin sensitivity, glucose and lipid metabolism. Therefore, SIRT1 is an attractive molecular target for the development of therapeutic treatments for many aspects of the metabolic syndrome including type 2 diabetes, mitochondrial diseases, atherosclerosis and dysregulated lipid homeostasis. SIRT1 activation can improve cardiac function through effects on multiple pathways including improved vasorelaxation, anti-inflammatory activity on macrophages and foam cell formation. Axon degeneration occurs frequently in neurodegenerative diseases such as Alzheimer's disease and amyotrophic lateral sclerosis. Both SIRT1 activators and overexpression of SIRT1 have been shown to slow *in vitro* cell death as well as *in vivo* neurodegeneration. SIRT1 activation also leads to anti-inflammatory activity through attenuation of NF-KB activity, the master regulator of innate immune responses.

It is the third objective of the present invention to provide compounds and/or pharmaceutically acceptable salts thereof which can be used as pharmaceutically active agents, especially for the treatment of cancer, metabolic, inflammatory, neurodegenerative or cardiovascular diseases as well as compositions comprising at least one of those compounds and/or pharmaceutically acceptable salts thereof as pharmaceutically active ingredients.

The objectives of the present invention are solved by the teaching of the independent claims. Further advantageous features, aspects and details of the invention are evident from the dependent claims, the description, the figures, and the examples of the present application.

### Brief description of the invention

### 1. LXRα agonists

Specific ligands for LXRs are important tools to combat atherosclerosis and cardiovascular diseases. The present invention discloses the new LXRα-specific agonist, compound **1**, which has a stilbenoide structure that is inspired from the natural LXR ligand oxysterol or sterols in general. These inventive compounds (I) thereby are useful for the treatment or prophylaxis of atherosclerosis or cardiovascular diseases.

### 2. SIRT1 inhibitor

SIRT1 inhibition plays a pivotal role in neurodegenerative diseases and cancer. In the present invention a SIRT 1 inhibitor (NP **1**), a terphenolide was identified and tested for its ability to increase p53 acetylation in human liver (HepG2) cells. The present invention discloses that the terphenolide compounds (II) have excellent biological activity against SIRT1. Thus, these inventive compounds (II) are useful for the treatment or prophylaxis of cancer or neurodegenerative diseases.

### 3. p300 inhibitor

p300 HAT has critical roles in a wide variety of cellular phenomena including cell cycle control, differentiation, and apoptosis. As p300 shows acetylation activity opposing SIRT1 deacetylation activity, health beneficial effects caused by potential SIRT1 activation could also be evoked by p300 inhibition. Therefore, as direct SIRT1 activation by small molecules is doubted, development of a p300 inhibitor could be useful for the treatment of diseases improved by potential SIRT1 activation. As the third aspect of the present invention the new p300 inhibitor (NP 9), a flavonol derivate was identified and tested for TNFα secretion in THP-1 cells. The present invention discloses that the flavonol derivates (III) are potent inhibitors against p300. Therefore, these inventive compounds (III) are useful for the treatment or prophylaxis of cancer, metabolic, inflammatory, neurodegenerative or cardiovascular diseases.

### Description of the invention

### 1. LXRα agonists

The present invention relates to stilbenoid compounds of the general formula (I) 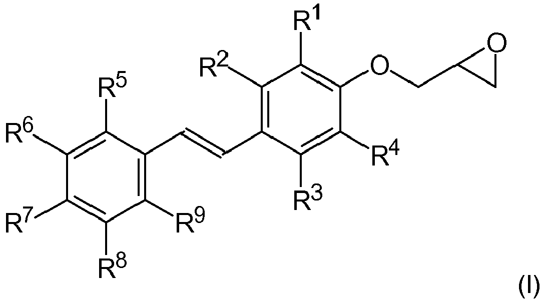 wherein
**R¹ - R⁹** represent independently of each other -H, -F, -Br, -Cl, -I, -CN, -NO₂, -**R³²**, -O**R³²**, -S**R³²**, -CH₂O**R³²**, -CO**R³²**, -CO₂**R³²**, -SO₂**R³²**, -N(**R³²**) **R³²**, -CON(**R³²**) **R³³**, -CH₂N(**R³²**) **R³³**, -N(**R³²**)SO₂**R³³** or - SO₂(**R³²**)N**R³³**; R³² and R³³ represent independently of each other -H, -CH₃, -C₂H₅, -CH=CH₂, -CH=CH, -CH₂-CH=CH₂, -C₃H₇, -CH(CH₃)₂, -cyclo-C₃H₅, -C₄H₉, -CH₂-CH(CH₃)₂, -CH(CH₃)-C₂H₅, -C(CH₃)₃, -CH₂F, -CHF₂, -CF₃, -CH₂F₃, or -C₂F₅;
and enantiomers, stereoisomeric forms, mixtures of enantiomers, diastereomers, mixtures of diastereomers, hydrates, solvates, acid salt forms, tautomers, and racemates of the above mentioned compounds and pharmaceutically acceptable salts thereof for use as an agonist of the LXRα protein.

Preferred is compound of general formula (I), wherein R¹ - R⁹ are -H. The chemical IUPAC name of this compound (compound 1) is 2-[[4-[(E)-styryl]phenoxy]-methyl]oxirane.

The other embodiment of the present invention is combination of at least one compound (I) and at least one additional LXRα agonist and preferred combination, wherein the at least one additional LXRα agonist is T0901317 (T09).

In other embodiment of the present invention compound (I) or said combination is for use as pharmaceutically active agent in medicine. Preferred, compound (I) or said combination is for use in the treatment or prophylaxis of atherosclerosis or cardiovascular diseases.

In another embodiment of the present invention compound (I) or said combination is for use a chemical tool for studying LXR biology.

In other embodiment, the present invention relates to pharmaceutical composition comprising at least one compound (I) as an active ingredient, together with at least one pharmaceutically acceptable other LXRα agonist, carrier, excipient and/or diluent. Preferred, pharmaceutically acceptable other LXRα agonist is T0901317 (T09).

The another embodiment is a nutraceutical composition containing at least one compound (I) or said combination, together with at least one nutraceutical acceptable carrier and/or excipient for use as food product, foodstuff, dietary supplement, nutritional supplement or a supplement composition for a food product or a foodstuff, in particular for the treatment or prophylaxis of atherosclerosis or cardiovascular diseases.

"A ligand" refers to a substance (usually a small molecule), that forms a complex with a biomolecule to serve a biological purpose. In a narrower sense, it is a signal triggering molecule, binding to a site on a target protein. The binding occurs by intermolecular forces, such as ionic bonds, hydrogen bonds and van der Waals forces. Ligand binding to a receptor (receptor protein) alters its chemical conformation. The conformational state of a receptor protein determines its functional state. Ligands include substrates, inhibitors, activators, and neurotransmitters. "An agonist" generally refers to a type of activator that binds and alters the activity of a receptor. "An inhibitor" is a ligand leading to inhibition of a receptor.

The liver X receptor α (LXRα) is a ligand-dependent nuclear receptor and the major regulator of reverse cholesterol transport in macrophages. This makes it an interesting target for treatment of atherosclerosis.

Many of LXR agonists have been described in the prior art. They are usually natural endogenous or exogenous molecules LXT binders, semi-synthetic or synthetic analogues, thereof, or new designed synthetic molecules. Examples of small molecule LXR agonists include GSK-2033, GSK-9772, GW3965, GW6340, F3-MethylAA, T0314407, T0901317 (T09), WYE-672 and oxystrerols such as 22(R)-hydroxycholesterol (22R), 24(S)-hydroxycholesterol, 24(S),25-epoxycholesterol and 27-hydroxycholesterol [Jakobsson T. et al. Trends Pharmacol Sci (2012), 33, 394-404]. Additional LXR agonists have been described in the prior art, e.g., WO 2012/079721, WO 2011/014661, US 2009/0118306, WO 2004/009091 and US 2002/0193357.

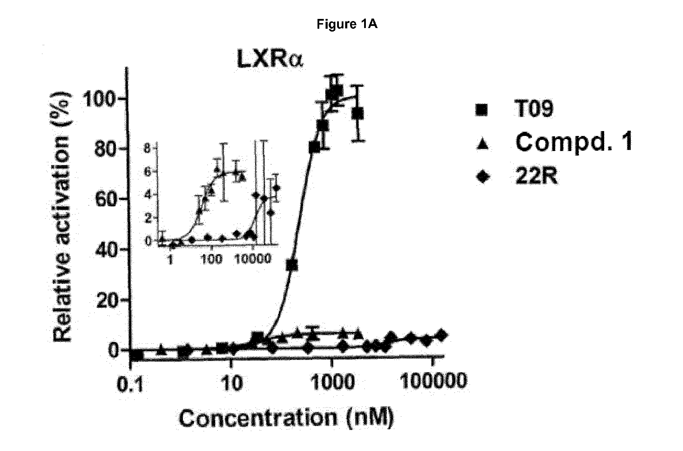

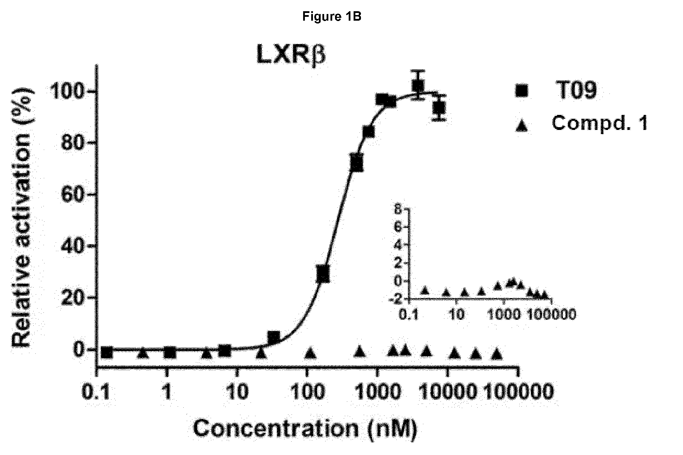

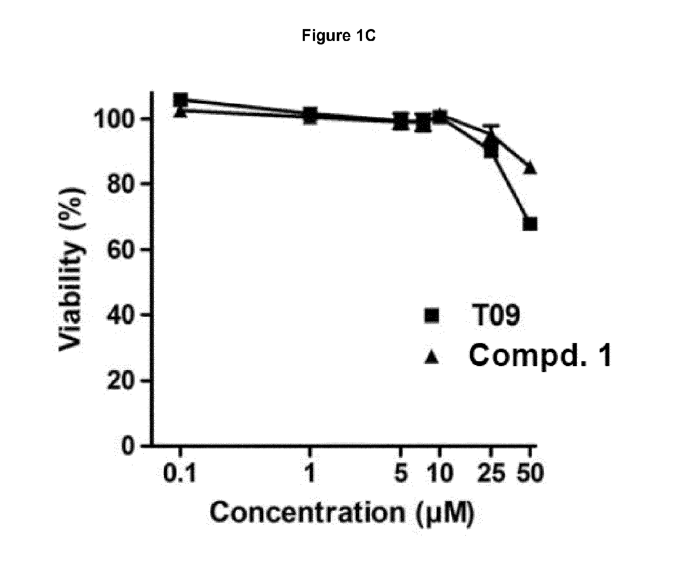

During the screening of a natural products library, the stilbenoid backbone is identified as a suitable candidate structure for activating LXR. Also an increased activation potential is observed by the addition of an epoxide moiety (compound **1**, Scheme 1). To analyse binding and activation ability of the new ligand compound **1** dual luciferase reporter-gene assays with LXRα and LXRβ ligand binding domains (LBD) in HEK-293 cells are applied (Figure 1 B and Figure 1 C). Whereas the control ligand T09 bound to LXRα and LXRβ with similar affinity, compound **1** showed characteristic specificity for LXRα (EC₅₀ = 35 nM) but less efficiently activated reporter gene transcription (6% vs. T09). In addition, the LXRα activation potential of the natural ligand 22R (22-R-Hydroxycholesterol) was measured (EC₅₀ = 13.3 µM, Figure 1. A)) and we observed even less efficiently activated reporter gene transcription of 4% vs. T09.

As for T09, viability assays revealed no cytotoxic effects of compound **1** up to 50 µM (Figure 1 D), making this compound suitable for further cell culture studies.

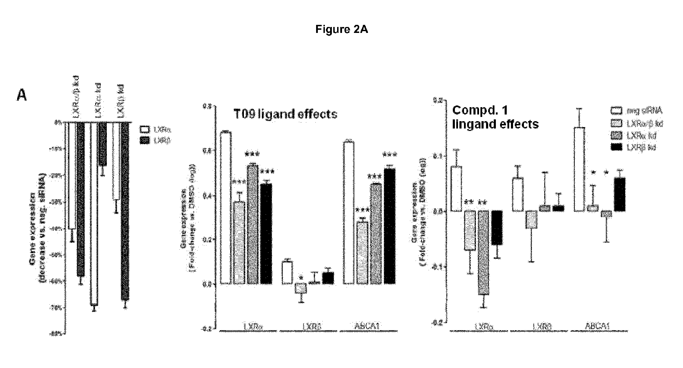

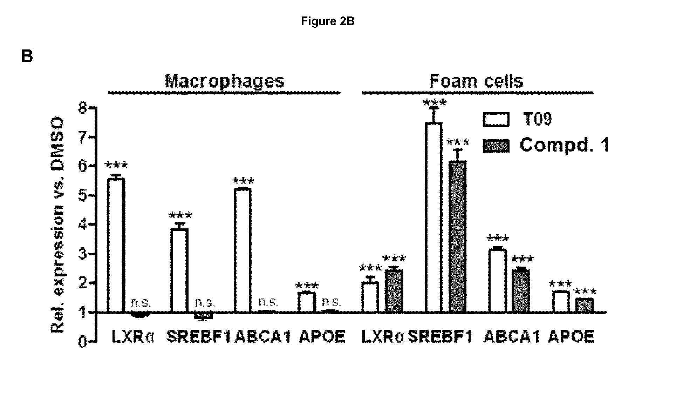

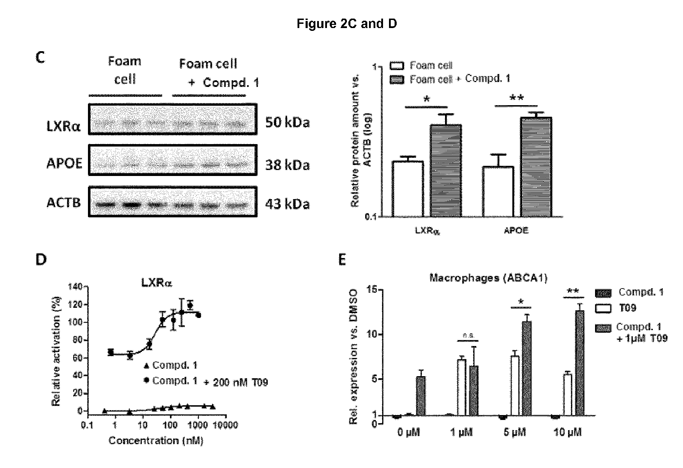

To test for ligand specificity LXRα and LXRβ specific knockdowns were done (Figure 2. A). For the T09 ligand in absence of both subtypes a significant reduction of LXRα and ABCA1 expression was observed, whereas compound **1** treatment showed a higher dependency on the LXRα subtype. Consistent with reporter gene assays a clear tendency towards higher LXRα than LXRβ specificity of compound **1** was observed.

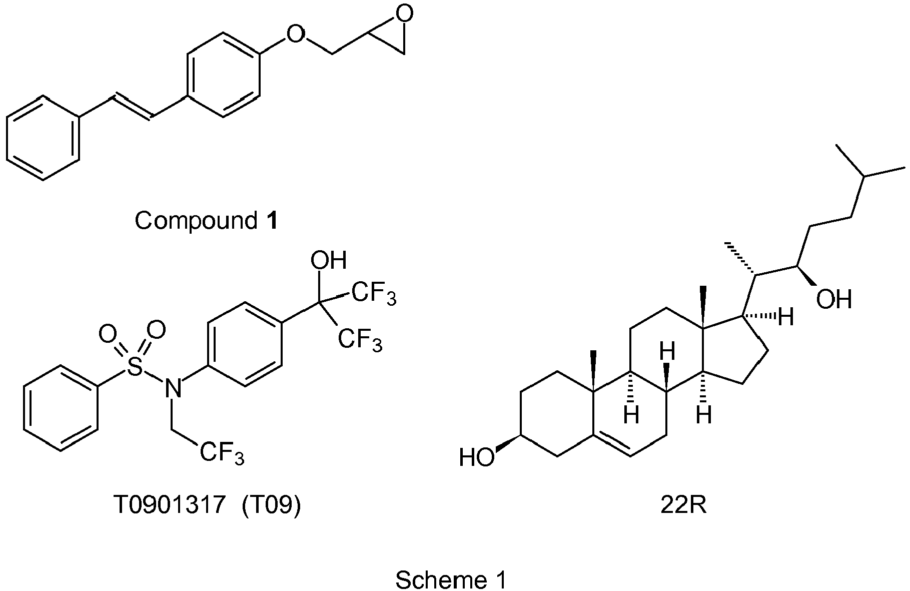

From analyses in the invention it is apparent that the unique conditional activation potential of compound **1** in diseased foam cells is highly dependent on a partner ligand. Reporter-gene assays and gene expression analyses have shown that compound **1** cooperates with the synthetic ligand T09 as well as with natural ligands derived from oxLDL in an additive manner. This effect could be explained by allosteric binding of compound **1** to the ligand binding domain. In addition it was assumed that a changed set of transcriptional LXR co-factors in foam cells could be recruited by compound **1** and thereby drive its specific actions. Due to its autoregulatory activation potential, LXRα features highly increased protein content in foam cells compared to macrophages. Strikingly, compound 1 only marginally activated LXR-target genes in macrophages but strongly in foam cells. Lipid loaded foam cells may provide a chemically favourable hydrophobic, fatty acids rich environment for compound 1. Furthermore, the level of LXRα in foam cells was about 10-fold higher than for LXRβ, suggesting that the observed effects of compound 1 were mainly driven by the alpha-subtype, which was confirmed in reporter gene and by trend in knockdown experiments (Figures 1. A-C and 2. A). These various mentioned effects can result in conditional activation of LXRα pathways in diseased foam cells due to compound 1 treatment.

For testing activation of LXRα in human macrophages and in diseased human foam cells, gene expression analyses were performed on its central target genes (namely *LXRα, SREBF1, ABCA1,* and *APOE* in Figure 2. B). In macrophages increased expression of LXRα target genes upon T09 treatment was observed, but not with compound **1**.

Strikingly, in foam cells compound **1** treatment resulted in potent target gene expression similar to T09, which was consistent with increased protein expression levels of LXRα target genes (Figure 2. C).

Using a competitive reporter-gene assay with a fixed concentration of T09 and increasing levels of compound **1**, additive effects on reporter gene activation (efficacy range 64%-111 %, Figure 2. D, Table 1) and *ABCA1* expression in macrophages (Figure 2. E) were detected, These data indicated conditional activation potency of compound **1** in foam cells, or the fact that activation of LXRα upon compound **1** treatment occurs selectively under the diseased condition of the foam cell.

**Table 1. Results from competitive reporter-gene assays.**

| Compound | **+DMSO** | | + **200 nM T0901317 (additive)** | |
|---|---|---|---|---|
| | EC₅₀ | Efficacy | EC₅₀ | Efficacy (range) |
| T09 | 239 nM | 100 % | | |
| 22R | 13.3 µM | 4% | | |
| compound **1** | 35 nM | 6% | 28 nM | 47 % (64 to 111 %) |

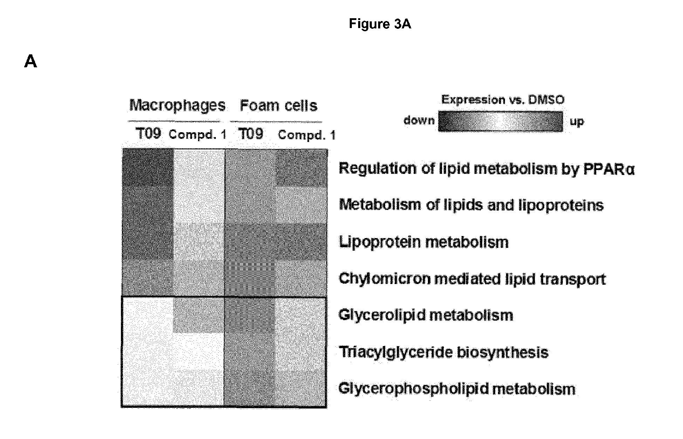

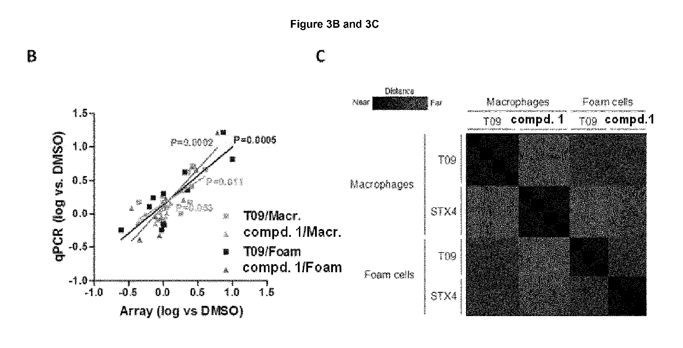

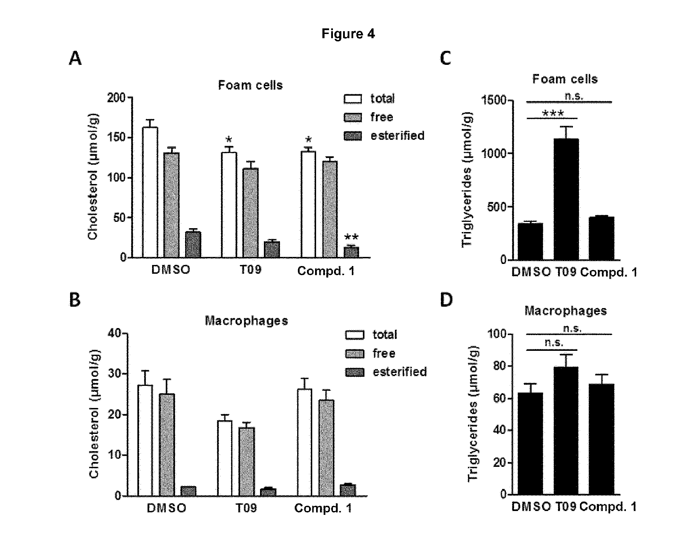

To study the genome-wide effects of compound 1 treatment microarray-based expression analyses were performed with compound 1, T09- or DMSO-treated macrophages and foam cells (Figure 3. A). Consistent with qPCR data (Figure 3. B), expression of genes involved in lipid metabolism processes was activated in T09-treated but not compound **1**-treated macrophages. However, in foam cells a high similarity between compound **1** and T09 treatment was detected (Figure 3. C). Lipid metabolism processes were comparably up-regulated by T09 and compound **1** in foam cells (Figure 3. A), and consequently led to a significant reduction of total and esterified cholesterol content in contrast to macrophage treatments (Figure 4. A and Figure 4. B).

Interestingly, a difference between T09 and compound **1** treatments in terms of triacylglyceride biosynthesis was noted. While this process was transcriptionally induced by T09 treatment, it was not regulated by compound **1** treatment in foam cells (Figure 3. A). Consequently, treatment with T09 but not compound **1** led to increased triglyceride levels in foam cells (Figure 4. C). In macrophages the triglyceride level stayed unchanged (Figure 4. D). Notably, despite the observed up-regulation of *SREBF1* mRNA (a common marker gene for lipogenesis) in compound **1**-treated cells (Figure 2. B), triglyceride levels were not increased in foam cells. Taken together the LXRα agonist compound **1** has a high potential to reduce excess cholesterol without undesirable increase in triglycerides in foam cells.

Consequently, in the present invention it is disclosed that combinatorial treatment with compound **1** and T09 resulted in an additive effect on reporter-gene activation and target gene expression. In physiological tests the cellular content of total and esterified cholesterol was significantly reduced by compound **1** without the undesirable increase in triglyceride levels as observed for T09.

In summary, compound **1** is a foam cell specific gene regulating molecule that showed no significant gene activation in normal macrophages. This new LXRα ligand can be applied as a chemical tool for investigating mechanistic aspects of macrophage homeostasis, and can be used as a lead structure for pharmaceutical development. The inventive stilbenoid compounds (I) as optimized compound **1** derivatives thereby are useful for the treatment or prophylaxis of atherosclerosis or cardiovascular diseases.

### Pharmaceutical compositions of stilbenoid (I)

The present invention also comprises pharmaceutically acceptable salts of the compounds according to the general formula (I), all stereoisomeric forms of the compounds according to the general formula (I) as well as solvates, especially hydrates or prodrugs thereof.

The compounds (I) of the present invention may form salts with organic or inorganic acids or bases. Examples of suitable acids for such acid addition salt formation are hydrochloric acid, hydrobromic acid, sulfuric acid, phosphoric acid, acetic acid, citric acid, oxalic acid, malonic acid, salicylic acid, p-aminosalicylic acid, malic acid, fumaric acid, succinic acid, ascorbic acid, maleic acid, sulfonic acid, phosphonic acid, perchloric acid, nitric acid, formic acid, propionic acid, gluconic acid, lactic acid, tartaric acid, hydroxymaleic acid, pyruvic acid, phenylacetic acid, benzoic acid, p-aminobenzoic acid, p-hydroxybenzoic acid, methanesulfonic acid, ethanesulfonic acid, nitrous acid, hydroxyethanesulfonic acid, ethylenesulfonic acid, p-toluenesulfonic acid, naphthylsulfonic acid, sulfanilic acid, camphorsulfonic acid, china acid, mandelic acid, o-methylmandelic acid, hydrogen-benzenesulfonic acid, picric acid, adipic acid, d-o-tolyltartaric acid, tartronic acid, (o, m, p)-toluic acid, naphthylamine sulfonic acid, and other mineral or carboxylic acids well known to those skilled in the art. The salts are prepared by contacting the free base form with a sufficient amount of the desired acid to produce a salt in the conventional manner.

In the case the inventive compounds (I) bear acidic groups, salts could also be formed with inorganic or organic bases. Examples for suitable inorganic or organic bases are, for example, NaOH, KOH, NH₄OH, tetraalkylammonium hydroxide, lysine or arginine and the like. Salts may be prepared in a conventional manner using methods well known in the art, for example by treatment of a solution of the compound of the general formula (I) with a solution of an acid, selected out of the group mentioned above.

Some of the compounds (I) of the present invention may be crystallised or recrystallised from solvents such as aqueous and organic solvents. In such cases solvates may be formed. This invention includes within its scope stoichiometric solvates including hydrates as well as compounds (I) containing variable amounts of water that may be produced by processes such as lyophilisation.

The compounds of the general formula (I) exist in the form of optical isomers, i.e. enantiomers and mixtures of said isomers in all ratios, e.g. racemic mixtures. The invention includes all such forms, in particular the pure isomeric forms or enantiomeric forms. The different isomeric forms may be separated or resolved one from the other by conventional methods, or any given isomer may be obtained by conventional synthetic methods or by stereospecific or asymmetric syntheses. Where a compound according to the general formula (I) contains an alkene moiety, the alkene can be presented as a cis or trans isomer or a mixture thereof. When an isomeric form of a compound of the invention is provided substantially free of other isomers, it will preferably contain less than 5% w/w, more preferably less than 2% w/w and especially less than 1 % w/w of the other isomer(s).

Another aspect of the present invention relates to the use of the inventive substituted stilbenoids as drugs, i.e. as pharmaceutically active agents applicable in medicine.

Surprisingly it was found that the above-mentioned stilbenoids as well as the pharmaceutical compositions comprising said stilbenoids are useful for treatment or prophylaxis of atherosclerosis, cardiovascular diseases, diabetes, or inflammatory diseases including skin diseases.

Thus, the stilbenoid compounds (I) of the present invention can be used for prophylaxis and treatment of atherosclerosis or cardiovascular diseases or for the preparation of a pharmaceutical formulation for prophylaxis and treatment of atherosclerosis or cardiovascular diseases.

Atherosclerosis also known as arteriosclerotic vascular disease or ASVD is a condition in which an artery wall thickens as a result of the accumulation of fatty materials such as cholesterol. It is asyndrome affecting arterial blood vessels, a chronic inflammatory response in the walls of arteries, caused largely by the accumulation of macrophage white blood cells and promoted by low-density lipoproteins (LDL, plasma proteins that carry cholesterol and triglycerides) without adequate removal of fats and cholesterol from the macrophages by functional high-density lipoproteins (HDL). It is commonly referred to as a hardening or furring of the arteries. It is caused by the formation of multiple plaques within the arteries.

Examples for cardiovascular diseases which can be treated and/or prevented by the inventive compounds (I) are selected from the group comprising or consisting of coronary heart disease, cardiomyopathy, hypertensive heart disease, heart failure, cor pulmonale, cardiac dysrhythmias, inflammatory heart disease, valvular heart disease, stroke and cerebrovascular disease and peripheral arterial disease.

Therefore, another aspect of the present invention is directed to pharmaceutical compositions comprising at least one compound (I) of the present invention as active ingredient, together with at least one pharmaceutically acceptable other LXR agonist (), carrier, excipient and/or diluents. The pharmaceutical compositions of the present invention can be prepared in a conventional solid or liquid carrier or diluent and a conventional pharmaceutically-made adjuvant at suitable dosage level in a known way. The preferred preparations are adapted for oral application. These administration forms include, for example, pills, tablets, film tablets, coated tablets, capsules, powders and deposits.

Furthermore, the present invention also includes pharmaceutical preparations for parenteral application, including dermal, intradermal, intragastral, intracutan, intravasal, intravenous, intramuscular, intraperitoneal, intranasal, intravaginal, intrabuccal, percutan, rectal, subcutaneous, sublingual, topical, or transdermal application, which preparations in addition to typical vehicles and/or diluents contain at least one compound (I) according to the present invention and/or a pharmaceutical acceptable salt thereof as active ingredient.

The pharmaceutical compositions according to the present invention containing at least one compound (I) according to the present invention, and/or a pharmaceutical acceptable salt thereof as active ingredient will typically be administered together with suitable carrier materials selected with respect to the intended form of administration, i.e. for oral administration in the form of tablets, capsules (either solid filled, semi-solid filled or liquid filled), powders for constitution, extrudates, deposits, gels, elixirs, dispersable granules, syrups, suspensions, and the like, and consistent with conventional pharmaceutical practices. For example, for oral administration in the form of tablets or capsules, the active drug component may be combined with any oral non-toxic pharmaceutically acceptable carrier, preferably with an inert carrier like lactose, starch, sucrose, cellulose, magnesium stearate, dicalcium phosphate, calcium sulfate, talc, mannitol, ethyl alcohol (liquid filled capsules) and the like. Moreover, suitable binders, lubricants, disintegrating agents and coloring agents may also be incorporated into the tablet or capsule. Powders and tablets may contain about 5 to about 95 weight % of the stilbenoid derived compound (I) and/or the respective pharmaceutically active salt as active ingredient.

Suitable binders include starch, gelatin, natural sugars, corn sweeteners, natural and synthetic gums such as acacia, sodium alginate, carboxymethylcellulose, polyethylene glycol and waxes. Among suitable lubricants there may be mentioned boric acid, sodium benzoate, sodium acetate, sodium chloride, and the like. Suitable disintegrants include starch, methylcellulose, guar gum, and the like. Sweetening and flavoring agents as well as preservatives may also be included, where appropriate. The disintegrants, diluents, lubricants, binders etc. are discussed in more detail below.

Moreover, the pharmaceutical compositions of the present invention may be formulated in sustained release form to provide the rate controlled release of any one or more of the components or active ingredients to optimise the therapeutic effect(s), e.g. antihistaminic activity and the like. Suitable dosage forms for sustained release include tablets having layers of varying disintegration rates or controlled release polymeric matrices impregnated with the active components and shaped in tablet form or capsules containing such impregnated or encapsulated porous polymeric matrices.

Liquid form preparations include solutions, suspensions, and emulsions. As an example, there may be mentioned water or water/propylene glycol solutions for parenteral injections or addition of sweeteners and opacifiers for oral solutions, suspensions, and emulsions. Liquid form preparations may also include solutions for intranasal administration. Aerosol preparations suitable for inhalation may include solutions and solids in powder form, which may be present in combination with a pharmaceutically acceptable carrier such as an inert, compressed gas, e.g. nitrogen. For preparing suppositories, a low melting fat or wax, such as a mixture of fatty acid glycerides like cocoa butter is melted first, and the active ingredient is then dispersed homogeneously therein e.g. by stirring. The molten, homogeneous mixture is then poured into conveniently sized moulds, allowed to cool, and thereby solidified.

Also included are solid form preparations which are intended to be converted, shortly before use, to liquid form preparations for either oral or parenteral administration. Such liquid forms include solutions, suspensions, and emulsions. The compounds (I) according to the present invention may also be delivered transdermally. The transdermal compositions may have the form of a cream, a lotion, an aerosol and/or an emulsion and may be included in a transdermal patch of the matrix or reservoir type as is known in the art for this purpose.

The term capsule as recited herein refers to a specific container or enclosure made e.g. of methyl cellulose, polyvinyl alcohols, or denatured gelatins or starch for holding or containing compositions comprising the active ingredient(s). Capsules with hard shells are typically made of blended of relatively high gel strength gelatins from bones or pork skin. The capsule itself may contain small amounts of dyes, opaquing agents, plasticisers and/or preservatives. Under tablet a compressed or moulded solid dosage form is understood which comprises the active ingredients with suitable diluents. The tablet may be prepared by compression of mixtures or granulations obtained by wet granulation, dry granulation, or by compaction well known to a person of ordinary skill in the art.

Oral gels refer to the active ingredients dispersed or solubilised in a hydrophilic semi-solid matrix. Powders for constitution refers to powder blends containing the active ingredients and suitable diluents which can be suspended e.g. in water or in juice.

Suitable diluents are substances that usually make up the major portion of the composition or dosage form. Suitable diluents include sugars such as lactose, sucrose, mannitol, and sorbitol, starches derived from wheat, corn rice, and potato, and celluloses such as microcrystalline cellulose. The amount of diluent in the composition can range from about 5 to about 95 % by weight of the total composition, preferably from about 25 to about 75 weight %, and more preferably from about 30 to about 60 weight %.

The term disintegrants refers to materials added to the composition to support break apart (disintegrate) and release the pharmaceutically active ingredients of a medicament. Suitable disintegrants include starches, "cold water soluble" modified starches such as sodium carboxymethyl starch, natural and synthetic gums such as locust bean, karaya, guar, tragacanth and agar, cellulose derivatives such as methylcellulose and sodium carboxymethylcellulose, microcrystalline celluloses, and cross-linked microcrystalline celluloses such as sodium croscaramellose, alginates such as alginic acid and sodium alginate, clays such as bentonites, and effervescent mixtures. The amount of disintegrant in the composition may range from about 2 to about 20 weight % of the composition, more preferably from about 5 to about 10 weight %.

Binders are substances which bind or "glue" together powder particles and make them cohesive by forming granules, thus serving as the "adhesive" in the formulation. Binders add cohesive strength already available in the diluent or bulking agent. Suitable binders include sugars such as sucrose, starches derived from wheat corn rice and potato, natural gums such as acacia, gelatin and tragacanth, derivatives of seaweed such as alginic acid, sodium alginate and ammonium calcium alginate, cellulose materials such as methylcellulose, sodium carboxymethylcellulose and hydroxypropylmethylcellulose, polyvinylpyrrolidone, and inorganic compounds such as magnesium aluminum silicate. The amount of binder in the composition may range from about 2 to about 20 weight % of the composition, preferably from about 3 to about 10 weight %, and more preferably from about 3 to about 6 weight %.

Lubricants refer to a class of substances which are added to the dosage form to enable the tablet granules etc. after being compressed to release from the mould or die by reducing friction or wear. Suitable lubricants include metallic stearates such as magnesium stearate, calcium stearate, or potassium stearate, stearic acid, high melting point waxes, and other water soluble lubricants such as sodium chloride, sodium benzoate, sodium acetate, sodium oleate, polyethylene glycols and D,L-leucine. Lubricants are usually added at the very last step before compression, since they must be present at the surface of the granules. The amount of lubricant in the composition may range from about 0.2 to about 5 weight % of the composition, preferably from about 0.5 to about 2 weight %, and more preferably from about 0.3 to about 1.5 weight % of the composition.

Glidents are materials that prevent caking of the components of the pharmaceutical composition and improve the flow characteristics of granulate so that flow is smooth and uniform. Suitable glidents include silicon dioxide and talc. The amount of glident in the composition may range from about 0.1 to about 5 weight % of the final composition, preferably from about 0.5 to about 2 weight %.

Coloring agents are excipients that provide coloration to the composition or the dosage form. Such excipients can include food grade dyes adsorbed onto a suitable adsorbent such as clay or aluminum oxide. The amount of the coloring agent may vary from about 0.1 to about 5 weight % of the composition, preferably from about 0.1 to about 1 weight %.

Said pharmaceutical compositions further comprise at least one active stilbenoid of the general formula (I).

### 2. SIRT1 inhibitor

Accordingly the present invention also relates to terphenolide compounds of the general formula (II) 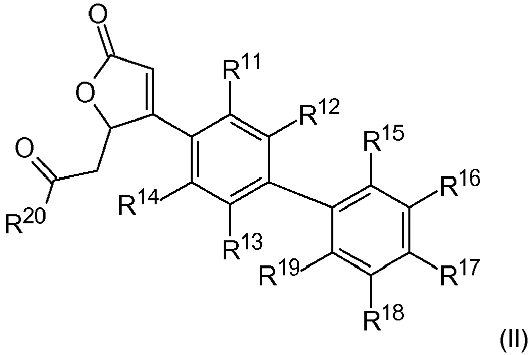 wherein
R¹¹ - R¹⁹ represent independently of each other -H, -F, -Br, -Cl, -I, -CN, -NO₂, -R³², -OR³², -SR³², -CH₂OR³², -COR³², -CO₂R³², -SO₂R³², -N(**R³²**) **R³²**, -CON(**R³²**) **R³³**, -CH₂N(**R³²**) **R³³**, -N(**R³²**)SO₂**R³³**, or - SO₂(**R³²**)N**R³³**;
**R²⁰** represents -O**R³²** or -N(**R³²**) **R³³**;
and enantiomers, stereoisomeric forms, mixtures of enantiomers, diastereomers, mixtures of diastereomers, hydrates, solvates, acid salt forms, tautomers, and racemates of the above mentioned compounds and pharmaceutically acceptable salts thereof for use as an inhibitor of the SIRT1 enzyme.

Preferred is compound of general formula (II), wherein **R¹¹** and **R¹³** are -OCH₃; **R¹⁴**, **R¹⁷** and **R²⁰** are -OH; , **R¹² R¹⁵**, **R¹⁶** , **R¹⁸** and **R¹⁹** are -H.

In other embodiment of the present invention compound (II) is for use as pharmaceutically active agent in medicine. Preferred, compound (II) is for use in the treatment or prophylaxis of neurodegenerative diseases or cancer.

More preferred, compound (II) is for use in the treatment or prophylaxis of cancer or neurodegenerative disease selected from Alzheimer's, Huntington's, respiratory diseases, lung cancer and colorectal cancer.

In other embodiment, the present invention relates to pharmaceutical composition comprising at least one compound (II) as an active ingredient, together with at least one pharmaceutically acceptable carrier, excipient and/or diluent.

the another embodiment is compound (II) for use a chemical tool for studying SIRT1 biology and preferred compound of general formula (II), wherein **R¹¹** and **R¹³** are - OCH₃; **R¹⁴**, **R¹⁷** and **R²⁰** are -OH; **R¹²**, **R¹⁵**, **R¹⁶** , **R¹⁸** and **R¹⁹** are -H.

The yet another embodiment is a nutraceutical composition containing at least one compound (II) together with at least one nutraceutical acceptable carrier and/or excipient for use as food product, foodstuff, dietary supplement, nutritional supplement or a supplement composition for a food product or a foodstuff, in particular for the treatment or prophylaxis of neurodegenerative diseases or cancer.

A library of 5500 natural products was screened for SIRT1 modulators. Therefore a MALDI-TOF MS based screening assay was applied. Whereas fluorescence based screening assays are prone to artifact generation, MALDI-TOF MS enables direct detection of peptide deacetylation by a 42 Da mass shift.

### SIRT1 inhibitors

Through this screening eight SIRT1 inhibitors were discovered (**NP 1 - 8**; Table 3), which were then tested for cytotoxicity in cell culture (Figure 13). Toxic compounds **(NP 6 - 8)** were excluded from further characterization. IC₅₀ values of the remaining five inhibitors (**NP 1 - 5**) were between 9.7 µM and 49 µM. SIRT1 inhibitors with such potency can have valuable biological effects [C. Gey et al. Angew Chem Int Ed Engl (2007), 46, 5219-5222].

**Table 3. SIRT1 Screening Hits**

| Compounds | Structure | IC₅₀ values (µM) |
|---|---|---|
| **NP** 1 | 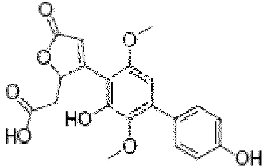 | 9.7 ± 0.7 |
| **NP2** | 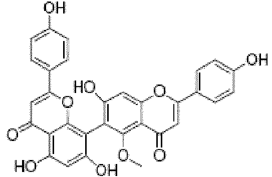 | 28±9 |
| **NP3** | 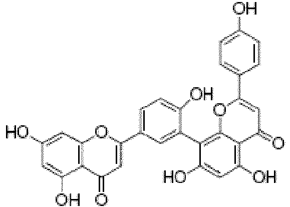 | 36±6 |
| **NP4** | 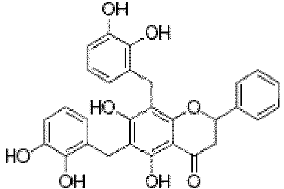 | 46 ± 4 |
| **NP 5** | 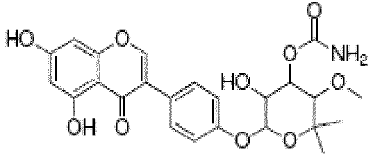 | 49±3 |
| **NP6** | 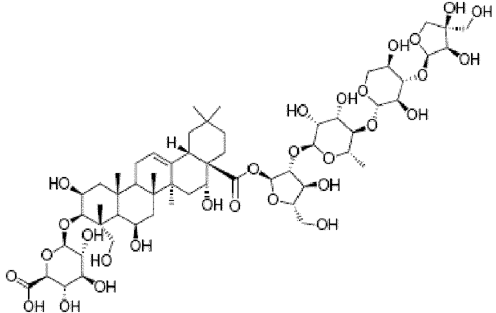 | n.d.^{[a]} |
| **NP7** | 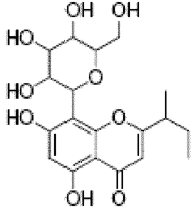 | n.d.^{[a]} |
| **NP 8** | 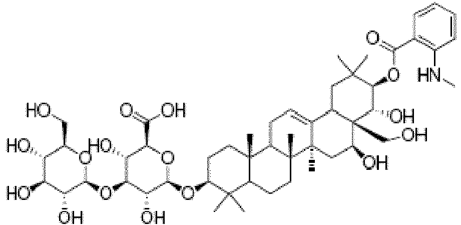 | n.d.^{[a]} |

| | | |
|---|---|---|
| [a] n.d.: not determined | | |

The chemical names of the hit compounds read as follows (Table 4):

**Table 4.**

| Compounds | Name |
|---|---|
| **NP** 1 | 2-(3-(3,4'-dihydroxy-2,5-dimethoxybiphenyl-4-yl)-5-oxo-2,5-dihydrofuran-2-yl)acetic acid |
| **NP 2** | 5',7,7'-trihydroxy-2,2'-bis(4-hydroxyphenyl)-5-methoxy-6,8'-bi(4H-chromene)-4,4'-dione |
| **NP 3** | 8-(5-(5,7-dihydroxy-4-oxo-4H-chromen-2-yl)-2-hydroxyphenyl)-5,7-dihydroxy-2-(4-hydroxyphenyl)-4H-chromen-4-one |
| **NP 4** | 6,8-bis(2,3-dihydroxybenzyl)-5,7-dihydroxy-2-phenylchroman-4-one |
| **NP 5** | 6-(4-(5,7-dihydroxy-4-oxo-4H-chromen-3-yl)phenoxy)-5-hydroxy-3-methoxy-2,2-dimethyl-tetrahydro-2H-pyran-4-yl carbamate |
| **NP 6** | (2S,3S,4S,5R,6R)-6-((2S,3R,4R,5R,6aR,6bS,8R,8aR,12aS,14bR)-8a-(((2S,3R,4S,5S)-3-((2S,3R,4S,5R,6S)-5-((2S,3R,4S,5R)-4-((2R,3R,4R)-3,4-dihydroxy-4-(hydroxymethyl)-tetrahydrofuran-2-yloxy)-3,5-dihydroxy-tetrahydro-2H-pyran-2-yloxy)-3,4-dihydroxy-6-methyl-tetrahydro-2H-pyran-2-yloxy)-4-hydroxy-5-(hydroxymethyl)-tetrahydrofuran-2-yloxy)carbonyl)-2,5,8-trihydroxy-4- |
| | (hydroxymethyl)-4,6a,6b,11,11,14b-hexamethyl-1,2,3,4,4a,5,6,6a,6b,7,8,8a,9,10,11,12,12a,14,14a,14b-icosahydropicen-3-yloxy)-3,4,5-trihydroxy-tetrahydro-2H-pyran-2-carboxylic acid |
| **NP 7** | 2-sec-butyl-5,7-dihydroxy-8-(3,4,5-trihydroxy-6-(hydroxymethyl)-tetrahydro-2H-pyran-2-yl)-4H-chromen-4-one |
| **NP 8** | (2S,3S,4S,5R,6R)-6-((3S,6aR,6bS,8S,8aR,9R,10R,14bR)-8,9-dihydroxy-8a-(hydroxymethyl)-4,4,6a,6b,11,11,14b-heptamethyl-10-(2-(methylamino)benzoyloxy)-1,2,3,4,4a,5,6,6a,6b,7,8,8a,9,10,11,12,12a,14,14a,14b-icosahydropicen-3-yloxy)-3,5-dihydroxy-4-((2S,3R,4S,5S,6R)-3,4,5-trihydroxy-6-(hydroxymethyl)-tetrahydro-2H-pyran-2-yloxy)-tetrahydro-2H-pyran-2-carboxylic acid |

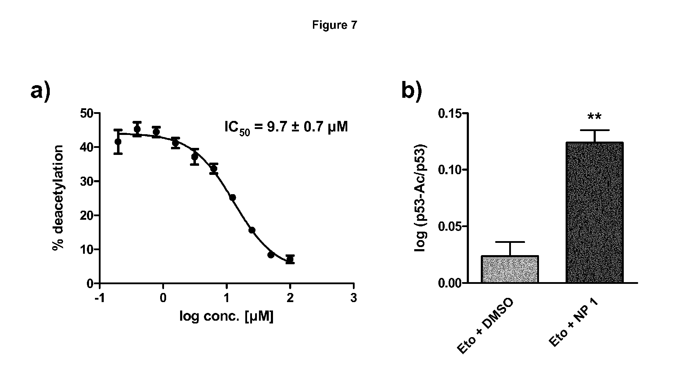

These primary hit compounds (NP **1** - **5**) could be used for the development of SIRT1 inhibitors. Most preferred, NP **1**, a terphenolide as lead structure could be further structurally optimized for the drug design of SIRT1 inhibitors, because NP **1** has already effective inhibitory activity (Figure 7. A).

The strongest inhibitor NP **1** was tested for its ability to increase p53 acetylation in human liver (HepG2) cells. Cells were treated for 16 hours with 30 µM of NP **1**. A significant increase of p53 acetylation in the nuclear fraction was observed by quantitative Western-Blot analysis (Figures 7. B and 8).

### Pharmaceutical applications of compound (II)

The present invention also comprises pharmaceutically acceptable salts of the compounds according to the general formula (II), all stereoisomeric forms of the compounds according to the general formula (II) as well as solvates, especially hydrates or prodrugs thereof.

The compounds (II) of the present invention may form salts with organic or inorganic acids or bases. Examples of suitable acids for such acid addition salt formation are hydrochloric acid, hydrobromic acid, sulfuric acid, phosphoric acid, acetic acid, citric acid, oxalic acid, malonic acid, salicylic acid, p-aminosalicylic acid, malic acid, fumaric acid, succinic acid, ascorbic acid, maleic acid, sulfonic acid, phosphonic acid, perchloric acid, nitric acid, formic acid, propionic acid, gluconic acid, lactic acid, tartaric acid, hydroxymaleic acid, pyruvic acid, phenylacetic acid, benzoic acid, p-aminobenzoic acid, p-hydroxybenzoic acid, methanesulfonic acid, ethanesulfonic acid, nitrous acid, hydroxyethanesulfonic acid, ethylenesulfonic acid, p-toluenesulfonic acid, naphthylsulfonic acid, sulfanilic acid, camphorsulfonic acid, china acid, mandelic acid, o-methylmandelic acid, hydrogen-benzenesulfonic acid, picric acid, adipic acid, d-o-tolyltartaric acid, tartronic acid, (o, m, p)-toluic acid, naphthylamine sulfonic acid, and other mineral or carboxylic acids well known to those skilled in the art. The salts are prepared by contacting the free base form with a sufficient amount of the desired acid to produce a salt in the conventional manner.

In the case the inventive compounds (II) bear acidic groups, salts could also be formed with inorganic or organic bases. Examples for suitable inorganic or organic bases are, for example, NaOH, KOH, NH₄OH, tetraalkylammonium hydroxide, lysine or arginine and the like. Salts may be prepared in a conventional manner using methods well known in the art, for example by treatment of a solution of the compound of the general formula (II) with a solution of an acid, selected out of the group mentioned above.

Some of the compounds (II) of the present invention may be crystallised or recrystallised from solvents such as aqueous and organic solvents. In such cases solvates may be formed. This invention includes within its scope stoichiometric solvates including hydrates as well as compounds containing variable amounts of water that may be produced by processes such as lyophilisation.

The compounds of the general formula (II) exist in the form of optical isomers, i.e. enantiomers and mixtures of said isomers in all ratios, e.g. racemic mixtures. The invention includes all such forms, in particular the pure isomeric forms or enantiomeric forms. The different isomeric forms may be separated or resolved one from the other by conventional methods, or any given isomer may be obtained by conventional synthetic methods or by stereospecific or asymmetric syntheses. Where a compound according to the general formula (II) contains an alkene moiety, the alkene can be presented as a cis or trans isomer or a mixture thereof. When an isomeric form of a compound of the invention is provided substantially free of other isomers, it will preferably contain less than 5% w/w, more preferably less than 2% w/w and especially less than 1 % w/w of the other isomer(s). Another aspect of the present invention relates to the use of the inventive substituted terphenolides (II) as chemical tools to understand the mode of action of SIRT1 and as drugs, i.e. as pharmaceutically active agents applicable in medicine.

Surprisingly it was found that the above-mentioned terphenolides(II) as well as the pharmaceutical compositions comprising said terphenolides (II) are useful for treatment or prophylaxis of cancer or neurodegenerative diseases.

Thus, the terphenolide compounds (II) of the present invention could be used for prophylaxis and treatment of cancer or neurodegenerative diseases or for the preparation of a pharmaceutical formulation for prophylaxis and treatment of cancer or neurodegenerative diseases.

Examples for cancer which can be treated and/or prevented by the inventive compounds (II) are selected from the group comprising or consisting of adenocarcinoma, choroidal melanoma, acute leukemia, acoustic neurinoma, ampullary carcinoma, anal carcinoma, astrocytoma, basal cell carcinoma, pancreatic cancer, desmoid tumor, bladder cancer, bronchial carcinoma, non-small cell lung cancer (NSCLC), breast cancer, Burkitt's lymphoma, corpus cancer, CUP-syndrome (carcinoma of unknown primary), colorectal cancer, small intestine cancer, small intestinal tumors, ovarian cancer, endometrial carcinoma, ependymoma, epithelial cancer types, Ewing's tumors, gastrointestinal tumors, gastric cancer, gallbladder cancer, gall bladder carcinomas, uterine cancer, cervical cancer, cervix, glioblastomas, gynecologic tumors, ear, nose and throat tumors, hematologic neoplasias, hairy cell leukemia, urethral cancer, skin cancer, skin testis cancer, brain tumors (gliomas), brain metastases, testicle cancer, hypophysis tumor, carcinoids, Kaposi's sarcoma, laryngeal cancer, germ cell tumor, bone cancer, colorectal carcinoma, head and neck tumors (tumors of the ear, nose and throat area), colon carcinoma, craniopharyngiomas, oral cancer (cancer in the mouth area and on lips), cancer of the central nervous system, liver cancer, liver metastases, leukemia, eyelid tumor, lung cancer, lymph node cancer (Hodgkin's/Non-Hodgkin's), lymphomas, stomach cancer, malignant melanoma, malignant neoplasia, malignant tumors gastrointestinal tract, breast carcinoma, rectal cancer, medulloblastomas, melanoma, meningiomas, Hodgkin's disease, mycosis fungoides, nasal cancer, neurinoma, neuroblastoma, kidney cancer, renal cell carcinomas, non-Hodgkin's lymphomas, oligodendroglioma, esophageal carcinoma, osteolytic carcinomas and osteoplastic carcinomas, osteosarcomas, ovarial carcinoma, pancreatic carcinoma, penile cancer, plasmocytoma, squamous cell carcinoma of the head and neck (SCCHN), prostate cancer, pharyngeal cancer, rectal carcinoma, retinoblastoma, vaginal cancer, thyroid carcinoma, Schneeberger disease, esophageal cancer, spinalioma, T-cell lymphoma (mycosis fungoides), thymoma, tube carcinoma, eye tumors, urethral cancer, urologic tumors, urothelial carcinoma, vulva cancer, wart appearance, soft tissue tumors, soft tissue sarcoma, Wilm's tumor, cervical carcinoma and tongue cancer.

Examples for neurodegenerative diseases which can be treated and/or prevented by the inventive compounds (II) are selected from the group comprising or consisting of Alzheimer's disease, Amyotrophic Lateral Sclerosis (ALS), Creutzfeldt-Jakob disease, Huntington's disease, Parkinson's disease, Primary progressive aphasia, Progressive supranuclear palsy.

Preferred, examples for cancer which can be treated and/or prevented by the inventive compounds (II) are selected from the group comprising or consisting of lung cancer and colorectal cancer.

Preferred, examples for neurodegenerative diseases which can be treated and/or prevented by the inventive compounds (II) are selected from the group comprising or consisting of Alzheimer's and Huntington's diseases.

Therefore, another aspect of the present invention is directed to pharmaceutical compositions comprising at least one compound (II) of the present invention as active ingredient, together with at least one pharmaceutically acceptable carrier, excipient and/or diluents. The pharmaceutical compositions of the present invention can be prepared in a conventional solid or liquid carrier or diluent and a conventional pharmaceutically-made adjuvant at suitable dosage level in a known way. The preferred preparations are adapted for oral application. These administration forms include, for example, pills, tablets, film tablets, coated tablets, capsules, powders and deposits.

Furthermore, the present invention also includes pharmaceutical preparations for parenteral application, including dermal, intradermal, intragastral, intracutan, intravasal, intravenous, intramuscular, intraperitoneal, intranasal, intravaginal, intrabuccal, percutan, rectal, subcutaneous, sublingual, topical, or transdermal application, which preparations in addition to typical vehicles and/or diluents contain at least one compound (II) according to the present invention and/or a pharmaceutical acceptable salt thereof as active ingredient.

The pharmaceutical compositions according to the present invention containing at least one compound (II) according to the present invention, and/or a pharmaceutical acceptable salt thereof as active ingredient will typically be administered together with suitable carrier materials selected with respect to the intended form of administration, i.e. for oral administration in the form of tablets, capsules (either solid filled, semi-solid filled or liquid filled), powders for constitution, extrudates, deposits, gels, elixirs, dispersable granules, syrups, suspensions, and the like, and consistent with conventional pharmaceutical practices. For example, for oral administration in the form of tablets or capsules, the active drug component may be combined with any oral non-toxic pharmaceutically acceptable carrier, preferably with an inert carrier like lactose, starch, sucrose, cellulose, magnesium stearate, dicalcium phosphate, calcium sulfate, talc, mannitol, ethyl alcohol (liquid filled capsules) and the like. Moreover, suitable binders, lubricants, disintegrating agents and coloring agents may also be incorporated into the tablet or capsule. Powders and tablets may contain about 5 to about 95 weight % of the terphenolide derived compound (II) and/or the respective pharmaceutically active salt as active ingredient.

Suitable binders include starch, gelatin, natural sugars, corn sweeteners, natural and synthetic gums such as acacia, sodium alginate, carboxymethylcellulose, polyethylene glycol and waxes. Among suitable lubricants there may be mentioned boric acid, sodium benzoate, sodium acetate, sodium chloride, and the like. Suitable disintegrants include starch, methylcellulose, guar gum, and the like. Sweetening and flavoring agents as well as preservatives may also be included, where appropriate. The disintegrants, diluents, lubricants, binders etc. are discussed in more detail below.

Moreover, the pharmaceutical compositions of the present invention may be formulated in sustained release form to provide the rate controlled release of any one or more of the components or active ingredients to optimise the therapeutic effect(s), e.g. antihistaminic activity and the like. Suitable dosage forms for sustained release include tablets having layers of varying disintegration rates or controlled release polymeric matrices impregnated with the active components and shaped in tablet form or capsules containing such impregnated or encapsulated porous polymeric matrices.

Liquid form preparations include solutions, suspensions, and emulsions. As an example, there may be mentioned water or water/propylene glycol solutions for parenteral injections or addition of sweeteners and opacifiers for oral solutions, suspensions, and emulsions. Liquid form preparations may also include solutions for intranasal administration. Aerosol preparations suitable for inhalation may include solutions and solids in powder form, which may be present in combination with a pharmaceutically acceptable carrier such as an inert, compressed gas, e.g. nitrogen. For preparing suppositories, a low melting fat or wax, such as a mixture of fatty acid glycerides like cocoa butter is melted first, and the active ingredient is then dispersed homogeneously therein e.g. by stirring. The molten, homogeneous mixture is then poured into conveniently sized moulds, allowed to cool, and thereby solidified.

Also included are solid form preparations which are intended to be converted, shortly before use, to liquid form preparations for either oral or parenteral administration. Such liquid forms include solutions, suspensions, and emulsions.

The compounds (II) according to the present invention may also be delivered transdermally. The transdermal compositions may have the form of a cream, a lotion, an aerosol and/or an emulsion and may be included in a transdermal patch of the matrix or reservoir type as is known in the art for this purpose.

The term capsule as recited herein refers to a specific container or enclosure made e.g. of methyl cellulose, polyvinyl alcohols, or denatured gelatins or starch for holding or containing compositions comprising the active ingredient(s). Capsules with hard shells are typically made of blended of relatively high gel strength gelatins from bones or pork skin. The capsule itself may contain small amounts of dyes, opaquing agents, plasticisers and/or preservatives. Under tablet a compressed or moulded solid dosage form is understood which comprises the active ingredients with suitable diluents. The tablet may be prepared by compression of mixtures or granulations obtained by wet granulation, dry granulation, or by compaction well known to a person of ordinary skill in the art.

Oral gels refer to the active ingredients dispersed or solubilised in a hydrophilic semi-solid matrix. Powders for constitution refers to powder blends containing the active ingredients and suitable diluents which can be suspended e.g. in water or in juice.

Suitable diluents are substances that usually make up the major portion of the composition or dosage form. Suitable diluents include sugars such as lactose, sucrose, mannitol, and sorbitol, starches derived from wheat, corn rice, and potato, and celluloses such as microcrystalline cellulose. The amount of diluent in the composition can range from about 5 to about 95 % by weight of the total composition, preferably from about 25 to about 75 weight %, and more preferably from about 30 to about 60 weight %.

The term disintegrants refers to materials added to the composition to support break apart (disintegrate) and release the pharmaceutically active ingredients of a medicament. Suitable disintegrants include starches, "cold water soluble" modified starches such as sodium carboxymethyl starch, natural and synthetic gums such as locust bean, karaya, guar, tragacanth and agar, cellulose derivatives such as methylcellulose and sodium carboxymethylcellulose, microcrystalline celluloses, and cross-linked microcrystalline celluloses such as sodium croscaramellose, alginates such as alginic acid and sodium alginate, clays such as bentonites, and effervescent mixtures. The amount of disintegrant in the composition may range from about 2 to about 20 weight % of the composition, more preferably from about 5 to about 10 weight %.

Binders are substances which bind or "glue" together powder particles and make them cohesive by forming granules, thus serving as the "adhesive" in the formulation. Binders add cohesive strength already available in the diluent or bulking agent. Suitable binders include sugars such as sucrose, starches derived from wheat corn rice and potato, natural gums such as acacia, gelatin and tragacanth, derivatives of seaweed such as alginic acid, sodium alginate and ammonium calcium alginate, cellulose materials such as methylcellulose, sodium carboxymethylcellulose and hydroxypropylmethylcellulose, polyvinylpyrrolidone, and inorganic compounds such as magnesium aluminum silicate. The amount of binder in the composition may range from about 2 to about 20 weight % of the composition, preferably from about 3 to about 10 weight %, and more preferably from about 3 to about 6 weight %.

Lubricants refer to a class of substances which are added to the dosage form to enable the tablet granules etc. after being compressed to release from the mould or die by reducing friction or wear. Suitable lubricants include metallic stearates such as magnesium stearate, calcium stearate, or potassium stearate, stearic acid, high melting point waxes, and other water soluble lubricants such as sodium chloride, sodium benzoate, sodium acetate, sodium oleate, polyethylene glycols and D,L-leucine. Lubricants are usually added at the very last step before compression, since they must be present at the surface of the granules. The amount of lubricant in the composition may range from about 0.2 to about 5 weight % of the composition, preferably from about 0.5 to about 2 weight %, and more preferably from about 0.3 to about 1.5 weight % of the composition.

Glidents are materials that prevent caking of the components of the pharmaceutical composition and improve the flow characteristics of granulate so that flow is smooth and uniform. Suitable glidents include silicon dioxide and talc. The amount of glident in the composition may range from about 0.1 to about 5 weight % of the final composition, preferably from about 0.5 to about 2 weight %.

Coloring agents are excipients that provide coloration to the composition or the dosage form. Such excipients can include food grade dyes adsorbed onto a suitable adsorbent such as clay or aluminum oxide. The amount of the coloring agent may vary from about 0.1 to about 5 weight % of the composition, preferably from about 0.1 to about 1 weight %.

Said pharmaceutical compositions further comprise at least one active terphenolide of the general formula (II).

### 3. p300 inhibitor: 8-prenylflavonol

The third aspect of the present invention is compounds that inhibit the activity of p300 and use thereof.

Accordingly the present invention further relates to compounds of the general formula (III) 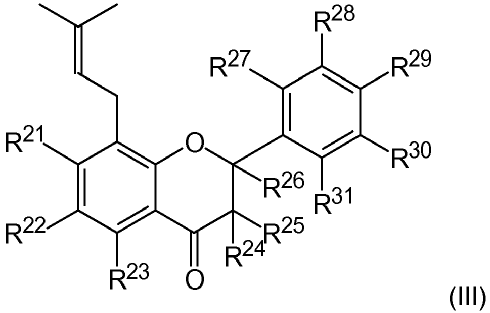 wherein
**R²¹** - **R²³** and **R²⁷** - **R³¹** represent independently of each other -H, -F, -Br, -Cl, -I, -CN, -NO₂, -**R³²**, -O**R³²**, -S**R³²**, -CH₂O**R³²**, -CO**R³²**, -CO₂**R³²**, -SO₂**R³²**, -N(**R³²**) **R³²** , -CON(**R³²**)**R³³**, -CH₂N(**R³²**) **R³³**, -N(**R³²**)SO₂**R³³**, or - SO₂(**R³²**)N**R³³**;
**R²⁴** - **R²⁶** represent independently of each other -H, -OH, or **R²⁵** and **R²⁶** form a C=C bond;
and enantiomers, stereoisomeric forms, mixtures of enantiomers, diastereomers, mixtures of diastereomers, hydrates, solvates, acid salt forms, tautomers, and racemates of the above mentioned compounds and pharmaceutically acceptable salts thereof for use as an inhibitor of the p300 protein.

Preferred is compound of general formula (III), wherein **R²²**, **R²⁷**, **R²⁸** and **R³¹** are -H; **R²¹**, **R²³** , **R²⁴**, **R²⁹** and **R³⁰** are -OH; **R²⁵** and **R²⁶** form a C=C bond.

In other embodiment of the present invention compound (III) is for use as pharmaceutically active agent in medicine. Preferred, compound (III) is for use in the treatment or prophylaxis of metabolic, inflammatory, cardiovascular, neurodegenerative diseases or cancer.

More preferred, compound (III) is for use in the treatment or prophylaxis of metabolic, inflammatory, cardiovascular, cancer or neurodegenerative disease selected from type 2 diabetes, atherosclerosis, optic neuritis, ALS, Alzheimer's, Huntington's, Parkinson's, ophthalmic, respiratory diseases, acute leukemia, pancreatic cancer, breast cancer, colorectal cancer, gastric cancer and ovarian cancer.

In other embodiment, the present invention relates to pharmaceutical composition comprising at least one compound (III) as an active ingredient, together with at least one pharmaceutically acceptable carrier, excipient and/or diluent.

The other embodiment is compound (III) for use a chemical tool for studying p300 biology and preferred compound of general formula (III), wherein **R²²**, **R²⁷**, **R²⁸** and **R³¹** are -H; **R²¹**, **R²³**, **R²⁴**, **R²⁹** and **R³⁰** are -OH; **R²⁵** and **R²⁶** form a C=C bond.

The yet another embodiment is a nutraceutical composition containing at least one compound (III) together with at least one nutraceutical acceptable carrier and/or excipient for use as food product, foodstuff, dietary supplement, nutritional supplement or a supplement composition for a food product or a foodstuff, in particular for the treatment or prophylaxis of metabolic, inflammatory, cardiovascular, neurodegenerative diseases or cancer.

### p300 inhibitors

The p300 has been implicated in a number of diverse biological functions including proliferation, cell cycle regulation, apoptosis, differentiation and DNA damage response. As p300 shows acetylation activity opposing SIRT1 deacetylation activity, health beneficial effects caused by potential SIRT1 activation could also be evoked by p300 inhibition. As direct SIRT1 activation by small molecules is doubted, development of a p300 inhibitor could be useful for the treatment of diseases improved by potential SIRT1 activation.

The above mentioned MALDI MS assay principle was adopted for detection of acetylation by the histone acetyltransferase (HAT) p300. Using this method, a novel p300 inhibitor (NP **9**) was identified and its IC₅₀ value of 1.71 µM ± 0.07 µM (Figure 17) was determined. This value was confirmed by applying a radioactivity based assay (2.1 µM ± 0.4 µM).
Hit compound, NP **9** has the following formula:

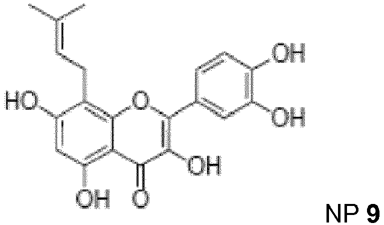

The chemical name of NP **9** is 2-(3,4-dihydroxyphenyl)-3,5,7-trihydroxy-8-(3-methylbut-2-enyl)-4H-chromen-4-one.

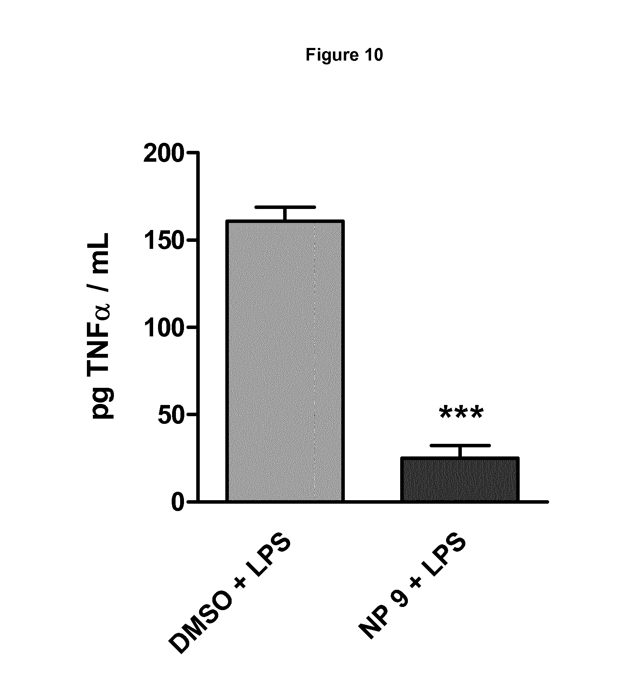

The flavonol (quercetin) derivative NP **9** is about 4 times stronger than anacardic acid (IC₅₀ 8.5 µM), found in the shell of cashew nuts [K. Balasubramanyam et al. J Biol Chem (2003), 278, 19134-19140], and about 3.5 times stronger than garcinol (IC₅₀ 7 µM), which is found in the fruit rind of a tropical evergreen tree [K. Balasubramanyam et al. J Biol Chem (2004), 279, 33716-33726].. In cell culture, NP **9** showed significant anti-inflammatory effects by reducing the levels of TNFα secreted by THP-1 cells (Figure 10). This is an interesting feature of p300 inhibitors, which was recently shown for the p300 inhibitor curcumin [J. M. Yun et al. J Nutr Biochem (2011), 22, 450-458]. It was furthermore shown for potential SIRT1 activators [V. M. Nayagam et al. J Biomol Screen (2006), 11 (8), 959-967], indicating similar functions of both p300 inhibition and SIRT1 activation.

This hit compound (NP **9**) could be used as lead structure for the further development of p300 inhibitors. The compounds of the general formula (III) optimized structurally could have better biological activity against p300 protein. These compounds can also be used as chemical tools to help understanding the mechanism of action of p300.

### Pharmaceutical applications of compound (III)

The present invention also comprises pharmaceutically acceptable salts of the compounds according to the general formula (III), all stereoisomeric forms of the compounds according to the general formula (III) as well as solvates, especially hydrates or prodrugs thereof.

The compounds (III) of the present invention may form salts with organic or inorganic acids or bases. Examples of suitable acids for such acid addition salt formation are hydrochloric acid, hydrobromic acid, sulfuric acid, phosphoric acid, acetic acid, citric acid, oxalic acid, malonic acid, salicylic acid, p-aminosalicylic acid, malic acid, fumaric acid, succinic acid, ascorbic acid, maleic acid, sulfonic acid, phosphonic acid, perchloric acid, nitric acid, formic acid, propionic acid, gluconic acid, lactic acid, tartaric acid, hydroxymaleic acid, pyruvic acid, phenylacetic acid, benzoic acid, p-aminobenzoic acid, p-hydroxybenzoic acid, methanesulfonic acid, ethanesulfonic acid, nitrous acid, hydroxyethanesulfonic acid, ethylenesulfonic acid, p-toluenesulfonic acid, naphthylsulfonic acid, sulfanilic acid, camphorsulfonic acid, china acid, mandelic acid, o-methylmandelic acid, hydrogen-benzenesulfonic acid, picric acid, adipic acid, d-o-tolyltartaric acid, tartronic acid, (o, m, p)-toluic acid, naphthylamine sulfonic acid, and other mineral or carboxylic acids well known to those skilled in the art. The salts are prepared by contacting the free base form with a sufficient amount of the desired acid to produce a salt in the conventional manner.

In the case the inventive compounds (III) bear acidic groups, salts could also be formed with inorganic or organic bases. Examples for suitable inorganic or organic bases are, for example, NaOH, KOH, NH₄OH, tetraalkylammonium hydroxide, lysine or arginine and the like. Salts may be prepared in a conventional manner using methods well known in the art, for example by treatment of a solution of the compound of the general formula (III) with a solution of an acid, selected out of the group mentioned above.

Some of the compounds of the present invention may be crystallised or recrystallised from solvents such as aqueous and organic solvents. In such cases solvates may be formed. This invention includes within its scope stoichiometric solvates including hydrates as well as compounds containing variable amounts of water that may be produced by processes such as lyophilisation.

The compounds of the general formula (III) exist in the form of optical isomers, i.e. enantiomers and mixtures of said isomers in all ratios, e.g. racemic mixtures. The invention includes all such forms, in particular the pure isomeric forms or enantiomeric forms. The different isomeric forms may be separated or resolved one from the other by conventional methods, or any given isomer may be obtained by conventional synthetic methods or by stereospecific or asymmetric syntheses. Where a compound according to the general formula (III) contains an alkene moiety, the alkene can be presented as a cis or trans isomer or a mixture thereof. When an isomeric form of a compound of the invention is provided substantially free of other isomers, it will preferably contain less than 5% w/w, more preferably less than 2% w/w and especially less than 1 % w/w of the other isomer(s).

Another aspect of the present invention relates to the use of the inventive substituted flavonols (III) as drugs, i.e. as pharmaceutically active agents applicable in medicine.

Surprisingly it was found that the above-mentioned flavonols as well as the pharmaceutical compositions comprising said flavonols are useful for treatment or prophylaxis of cancer, metabolic, inflammatory, neurodegenerative or cardiovascular diseases.

Thus, the flavonol compounds (III) of the present invention can be used for prophylaxis and treatment of cancer, metabolic, inflammatory, neurodegenerative or cardiovascular diseases or for the preparation of a pharmaceutical formulation for prophylaxis and treatment of cancer, metabolic, inflammatory, neurodegenerative or cardiovascular diseases.

Examples for cardiovascular diseases which can be treated and/or prevented by the inventive compounds of the formula (III) are selected from the group comprising or consisting of coronary heart disease, cardiomyopathy, hypertensive heart disease, heart failure, cor pulmonale, cardiac dysrhythmias, inflammatory heart disease, valvular heart disease, stroke and cerebrovascular disease and peripheral arterial disease.

Examples for cancer types which can be treated and/or prevented by the inventive compounds of the formula (III) are selected from the group comprising or consisting of adenocarcinoma, choroidal melanoma, acute leukemia, acoustic neurinoma, ampullary carcinoma, anal carcinoma, astrocytoma, basal cell carcinoma, pancreatic cancer, desmoid tumor, bladder cancer, bronchial carcinoma, non-small cell lung cancer (NSCLC), breast cancer, Burkitt's lymphoma, corpus cancer, CUP-syndrome (carcinoma of unknown primary), colorectal cancer, small intestine cancer, small intestinal tumors, ovarian cancer, endometrial carcinoma, ependymoma, epithelial cancer types, Ewing's tumors, gastrointestinal tumors, gastric cancer, gallbladder cancer, gall bladder carcinomas, uterine cancer, cervical cancer, cervix, glioblastomas, gynecologic tumors, ear, nose and throat tumors, hematologic neoplasias, hairy cell leukemia, urethral cancer, skin cancer, skin testis cancer, brain tumors (gliomas), brain metastases, testicle cancer, hypophysis tumor, carcinoids, Kaposi's sarcoma, laryngeal cancer, germ cell tumor, bone cancer, colorectal carcinoma, head and neck tumors (tumors of the ear, nose and throat area), colon carcinoma, craniopharyngiomas, oral cancer (cancer in the mouth area and on lips), cancer of the central nervous system, liver cancer, liver metastases, leukemia, eyelid tumor, lung cancer, lymph node cancer (Hodgkin's/Non-Hodgkin's), lymphomas, stomach cancer, malignant melanoma, malignant neoplasia, malignant tumors gastrointestinal tract, breast carcinoma, rectal cancer, medulloblastomas, melanoma, meningiomas, Hodgkin's disease, mycosis fungoides, nasal cancer, neurinoma, neuroblastoma, kidney cancer, renal cell carcinomas, non-Hodgkin's lymphomas, oligodendroglioma, esophageal carcinoma, osteolytic carcinomas and osteoplastic carcinomas, osteosarcomas, ovarial carcinoma, pancreatic carcinoma, penile cancer, plasmocytoma, squamous cell carcinoma of the head and neck (SCCHN), prostate cancer, pharyngeal cancer, rectal carcinoma, retinoblastoma, vaginal cancer, thyroid carcinoma, Schneeberger disease, esophageal cancer, spinalioma, T-cell lymphoma (mycosis fungoides), thymoma, tube carcinoma, eye tumors, urethral cancer, urologic tumors, urothelial carcinoma, vulva cancer, wart appearance, soft tissue tumors, soft tissue sarcoma, Wilm's tumor, cervical carcinoma and tongue cancer.

Examples for metabolic diseases which can be treated and/or prevented by the inventive compounds of the formula (III) are selected from the group comprising or consisting of Hyperthyroidism, Hypothyroidism, Diabetes, Addison's Disease, GM2 Galactosemia, Gangliosidosis, Dyslipidemia, Hipolipidemia, Glucose-6-Phosphate Dehydrogenase Deficiency, Phenylketonuria.

Examples for inflammatory diseases which can be treated and/or prevented by the inventive compounds of the formula (III) are selected from the group comprising or consisting of acne vulgaris, ankylosing spondylitis, arthritis (osteoarthritis, rheumatoid arthritis (RA), psoriatic arthritis), asthma, atherosclerosis, autoimmune Diseases, celiac disease, chronic prostatitis, colitis, Crohn'S disease, dermatitis, diverticulitis, glomerulonephritis, hepatitis, hypersensitivities, inflammatory bowel diseases, interstitial Cystitis, irritable bowel syndrome (IBS), lupus erythematous, nephritis, optic neuritis, ophthalmic diseases, respiratory diseases, pelvic inflammatory disease, reperfusion injury, rheumatoid arthritis, sarcoidosis, transplant rejection, ulcerative colitis, vasculitis.

Examples for neurodegenerative diseases which can be treated and/or prevented by the inventive compounds of the formula (III) are selected from the group comprising or consisting of Alzheimer's disease, Amyotrophic Lateral Sclerosis (ALS), Creutzfeldt-Jakob disease, Huntington's disease, Parkinson's disease, Primary progressive aphasia, Progressive supranuclear palsy.

Preferred, examples for metabolic diseases which can be treated and/or prevented by the inventive compounds of the formula (III) are type 2 diabetes.

Preferred, examples for inflammatory diseases which can be treated and/or prevented by the inventive compounds of the formula (III) are atherosclerosis, optic neuritis, ophthalmic diseases and respiratory diseases.

Preferred, examples for cancers which can be treated and/or prevented by the inventive compounds of the formula (III) are acute leukemia, pancreatic cancer, breast cancer, colorectal cancer, gastric cancer and ovarian cancer.

Preferred, examples for neurodegenerative diseases which can be treated and/or prevented by the inventive compounds of the formula (III) are ALS, Alzheimer's, Huntington's, and Parkinson's disease.

More preferred, the aforementioned cardiovascular diseases can be treated and/or prevented by the inventive compounds of the formula (III).

Therefore, another aspect of the present invention is directed to pharmaceutical compositions comprising at least one compound (III) of the present invention as active ingredient, together with at least one pharmaceutically acceptable carrier, excipient and/or diluents. The pharmaceutical compositions of the present invention can be prepared in a conventional solid or liquid carrier or diluent and a conventional pharmaceutically-made adjuvant at suitable dosage level in a known way. The preferred preparations are adapted for oral application. These administration forms include, for example, pills, tablets, film tablets, coated tablets, capsules, powders and deposits.

Furthermore, the present invention also includes pharmaceutical preparations for parenteral application, including dermal, intradermal, intragastral, intracutan, intravasal, intravenous, intramuscular, intraperitoneal, intranasal, intravaginal, intrabuccal, percutan, rectal, subcutaneous, sublingual, topical, or transdermal application, which preparations in addition to typical vehicles and/or diluents contain at least one compound (III) according to the present invention and/or a pharmaceutical acceptable salt thereof as active ingredient.

The pharmaceutical compositions according to the present invention containing at least one compound (III) according to the present invention, and/or a pharmaceutical acceptable salt thereof as active ingredient will typically be administered together with suitable carrier materials selected with respect to the intended form of administration, i.e. for oral administration in the form of tablets, capsules (either solid filled, semi-solid filled or liquid filled), powders for constitution, extrudates, deposits, gels, elixirs, dispersable granules, syrups, suspensions, and the like, and consistent with conventional pharmaceutical practices. For example, for oral administration in the form of tablets or capsules, the active drug component may be combined with any oral non-toxic pharmaceutically acceptable carrier, preferably with an inert carrier like lactose, starch, sucrose, cellulose, magnesium stearate, dicalcium phosphate, calcium sulfate, talc, mannitol, ethyl alcohol (liquid filled capsules) and the like. Moreover, suitable binders, lubricants, disintegrating agents and coloring agents may also be incorporated into the tablet or capsule. Powders and tablets may contain about 5 to about 95 weight % of the flavonol derived compound (III) and/or the respective pharmaceutically active salt as active ingredient.

Suitable binders include starch, gelatin, natural sugars, corn sweeteners, natural and synthetic gums such as acacia, sodium alginate, carboxymethylcellulose, polyethylene glycol and waxes. Among suitable lubricants there may be mentioned boric acid, sodium benzoate, sodium acetate, sodium chloride, and the like. Suitable disintegrants include starch, methylcellulose, guar gum, and the like. Sweetening and flavoring agents as well as preservatives may also be included, where appropriate. The disintegrants, diluents, lubricants, binders etc. are discussed in more detail below.

Moreover, the pharmaceutical compositions of the present invention may be formulated in sustained release form to provide the rate controlled release of any one or more of the components or active ingredients to optimise the therapeutic effect(s), e.g. antihistaminic activity and the like. Suitable dosage forms for sustained release include tablets having layers of varying disintegration rates or controlled release polymeric matrices impregnated with the active components and shaped in tablet form or capsules containing such impregnated or encapsulated porous polymeric matrices.

Liquid form preparations include solutions, suspensions, and emulsions. As an example, there may be mentioned water or water/propylene glycol solutions for parenteral injections or addition of sweeteners and opacifiers for oral solutions, suspensions, and emulsions. Liquid form preparations may also include solutions for intranasal administration. Aerosol preparations suitable for inhalation may include solutions and solids in powder form, which may be present in combination with a pharmaceutically acceptable carrier such as an inert, compressed gas, e.g. nitrogen. For preparing suppositories, a low melting fat or wax, such as a mixture of fatty acid glycerides like cocoa butter is melted first, and the active ingredient is then dispersed homogeneously therein e.g. by stirring. The molten, homogeneous mixture is then poured into conveniently sized moulds, allowed to cool, and thereby solidified.

Also included are solid form preparations which are intended to be converted, shortly before use, to liquid form preparations for either oral or parenteral administration. Such liquid forms include solutions, suspensions, and emulsions.

The compounds according to the present invention may also be delivered transdermally. The transdermal compositions may have the form of a cream, a lotion, an aerosol and/or an emulsion and may be included in a transdermal patch of the matrix or reservoir type as is known in the art for this purpose.

The term capsule as recited herein refers to a specific container or enclosure made e.g. of methyl cellulose, polyvinyl alcohols, or denatured gelatins or starch for holding or containing compositions comprising the active ingredient(s). Capsules with hard shells are typically made of blended of relatively high gel strength gelatins from bones or pork skin. The capsule itself may contain small amounts of dyes, opaquing agents, plasticisers and/or preservatives. Under tablet a compressed or moulded solid dosage form is understood which comprises the active ingredients with suitable diluents. The tablet may be prepared by compression of mixtures or granulations obtained by wet granulation, dry granulation, or by compaction well known to a person of ordinary skill in the art.

Oral gels refer to the active ingredients dispersed or solubilised in a hydrophilic semi-solid matrix. Powders for constitution refers to powder blends containing the active ingredients and suitable diluents which can be suspended e.g. in water or in juice.

Suitable diluents are substances that usually make up the major portion of the composition or dosage form. Suitable diluents include sugars such as lactose, sucrose, mannitol, and sorbitol, starches derived from wheat, corn rice, and potato, and celluloses such as microcrystalline cellulose. The amount of diluent in the composition can range from about 5 to about 95 % by weight of the total composition, preferably from about 25 to about 75 weight %, and more preferably from about 30 to about 60 weight %.

The term disintegrants refers to materials added to the composition to support break apart (disintegrate) and release the pharmaceutically active ingredients of a medicament. Suitable disintegrants include starches, "cold water soluble" modified starches such as sodium carboxymethyl starch, natural and synthetic gums such as locust bean, karaya, guar, tragacanth and agar, cellulose derivatives such as methylcellulose and sodium carboxymethylcellulose, microcrystalline celluloses, and cross-linked microcrystalline celluloses such as sodium croscaramellose, alginates such as alginic acid and sodium alginate, clays such as bentonites, and effervescent mixtures. The amount of disintegrant in the composition may range from about 2 to about 20 weight % of the composition, more preferably from about 5 to about 10 weight %.

Binders are substances which bind or "glue" together powder particles and make them cohesive by forming granules, thus serving as the "adhesive" in the formulation. Binders add cohesive strength already available in the diluent or bulking agent. Suitable binders include sugars such as sucrose, starches derived from wheat corn rice and potato, natural gums such as acacia, gelatin and tragacanth, derivatives of seaweed such as alginic acid, sodium alginate and ammonium calcium alginate, cellulose materials such as methylcellulose, sodium carboxymethylcellulose and hydroxypropylmethylcellulose, polyvinylpyrrolidone, and inorganic compounds such as magnesium aluminum silicate. The amount of binder in the composition may range from about 2 to about 20 weight % of the composition, preferably from about 3 to about 10 weight %, and more preferably from about 3 to about 6 weight %.

Lubricants refer to a class of substances which are added to the dosage form to enable the tablet granules etc. after being compressed to release from the mould or die by reducing friction or wear. Suitable lubricants include metallic stearates such as magnesium stearate, calcium stearate, or potassium stearate, stearic acid, high melting point waxes, and other water soluble lubricants such as sodium chloride, sodium benzoate, sodium acetate, sodium oleate, polyethylene glycols and D,L-leucine. Lubricants are usually added at the very last step before compression, since they must be present at the surface of the granules. The amount of lubricant in the composition may range from about 0.2 to about 5 weight % of the composition, preferably from about 0.5 to about 2 weight %, and more preferably from about 0.3 to about 1.5 weight % of the composition.

Glidents are materials that prevent caking of the components of the pharmaceutical composition and improve the flow characteristics of granulate so that flow is smooth and uniform. Suitable glidents include silicon dioxide and talc. The amount of glident in the composition may range from about 0.1 to about 5 weight % of the final composition, preferably from about 0.5 to about 2 weight %.

Coloring agents are excipients that provide coloration to the composition or the dosage form. Such excipients can include food grade dyes adsorbed onto a suitable adsorbent such as clay or aluminum oxide. The amount of the coloring agent may vary from about 0.1 to about 5 weight % of the composition, preferably from about 0.1 to about 1 weight %.

Said pharmaceutical compositions further comprise at least one active flavonol of the general formula (III).

### Nutraceutical applications of compounds (I), (II) and (III)

In yet another aspect, the invention relates to a nutraceutical composition according to the invention, in particular a food or beverage or a supplement composition for a food or beverage further comprising at least one nutraceutically acceptable carrier and/or excipients.

The term nutraceutical composition as used herein include food product, foodstuff, dietary supplement, nutritional supplement or a supplement composition for a food product or a foodstuff.

Thus, in another embodiment the present invention relates to a nutraceutical composition comprising at least one compound or said combination of according to the invention with the definitions and preferences as given above wherein the nutraceutical is a food product, foodstuff, dietary supplement, nutritional supplement or a supplement composition for a food product or a foodstuff preferably a dietary supplement, a nutritional supplement or a supplement composition for a food or a foodstuff, preferably a supplement, nutritional supplement or a supplement composition for a food product or a foodstuff.

The preferred embodiment is a nutraceutical composition containing at least one compound (I) or said combination of at least one compound (I) and at least one additional LXRα agonist, together with at least one nutraceutical acceptable carrier and/or excipient for use as food product, foodstuff, dietary supplement, nutritional supplement or a supplement composition for a food product or a foodstuff, in particular for the treatment or prophylaxis of atherosclerosis or cardiovascular diseases.

The further preferred embodiment is a nutraceutical composition containing at least one compound (II) together with at least one nutraceutical acceptable carrier and/or excipient for use as food product, foodstuff, dietary supplement, nutritional supplement or a supplement composition for a food product or a foodstuff, in particular for the treatment or prophylaxis of neurodegenerative diseases or cancer.

The further preferred embodiment is a nutraceutical composition containing at least one compound (III) together with at least one nutraceutical acceptable carrier and/or excipient for use as food product, foodstuff, dietary supplement, nutritional supplement or a supplement composition for a food product or a foodstuff, in particular for the treatment or prophylaxis of metabolic, inflammatory, cardiovascular, neurodegenerative diseases or cancer.

As used herein, the term "food product" refers to any food or feed suitable for consumption by humans or animals. The food product may be a prepared and packaged food (e.g., mayonnaise, salad dressing, bread, or cheese food) or an animal feed (e.g., extruded and pelleted animal feed, coarse mixed feed or pet food composition). As used herein, the term "foodstuff" refers to any substance fit for human or animal consumption. The term "dietary supplement" refers to a small amount of a compound for supplementation of a human or animal diet packaged in single or multiple dose units. Dietary supplements do not generally provide significant amounts of calories but may contain other micronutrients (e.g., vitamins or minerals). The term "nutritional supplement" refers to a composition comprising a dietary supplement in combination with a source of calories. In some embodiments, nutritional supplements are meal replacements or supplements (e.g., nutrient or energy bars or nutrient beverages or concentrates).

Food products or foodstuffs are for example beverages such as non-alcoholic and alcoholic drinks as well as liquid preparation to be added to drinking water and liquid food, non-alcoholic drinks are for instance soft drinks, sport drinks, fruit juices, such as for example orange juice, apple juice and grapefruit juice; lemonades, teas, near-water drinks and milk and other dairy drinks such as for example yoghurt drinks, and diet drinks. In another embodiment food products or foodstuffs refer to solid or semi-solid foods comprising the composition according to the invention. These forms can include, but are not limited to baked goods such as cakes and cookies, puddings, dairy products, confections, snack foods, or frozen confections or novelties (e.g., ice cream, milk shakes), prepared frozen meals, candy, snack products (e.g., chips), liquid food such as soups, spreads, sauces, salad dressings, prepared meat products, cheese, yogurt and any other fat or oil containing foods, and food ingredients (e.g., wheat flour).

The term food products or foodstuffs also includes functional foods and prepared food products, the latter referring to any pre-packaged food approved for human consumption.

Animal feed including pet food compositions advantageously include food intended to supply necessary dietary requirements, as well as treats (e.g., dog biscuits) or other food supplements. The animal feed comprising the composition according to the invention may be in the form of a dry composition (for example, kibble), semi-moist composition, wet composition, or any mixture thereof. Alternatively or additionally, the animal feed is a supplement, such as a gravy, drinking water, yogurt, powder, suspension, chew, treat (e.g., biscuits) or any other delivery form.

Dietary supplements of the present invention may be delivered in any suitable format. In preferred embodiments, dietary supplements are formulated for oral delivery. The ingredients of the dietary supplement of this invention are contained in acceptable excipients and/or carriers for oral consumption. The actual form of the carrier, and thus, the dietary supplement itself, is not critical.

The carrier may be a liquid, gel, gelcap, capsule, powder, solid tablet (coated or non-coated), tea, or the like. The dietary supplement is preferably in the form of a tablet or capsule and most preferably in the form of a hard (shell) gelatin capsule. Suitable excipient and/or carriers include maltodextrin, calcium carbonate, dicalcium phosphate, tricalcium phosphate, microcrystalline cellulose, dextrose, rice flour, magnesium stearate, stearic acid, croscarmellose sodium, sodium starch glycolate, crospovidone, sucrose, vegetable gums, lactose, methylcellulose, povidone, carboxymethylcellulose, com starch, and the like (including mixtures thereof). Preferred carriers include calcium carbonate, magnesium stearate, maltodextrin, and mixtures thereof. The various ingredients and the excipient and/or carrier are mixed and formed into the desired form using conventional techniques. The tablet or capsule of the present invention may be coated with an enteric coating that dissolves at a pH of about 6.0 to 7.0. A suitable enteric coating that dissolves in the small intestine but not in the stomach is cellulose acetate phthalate. Further details on techniques for formulation for and administration may be found in the latest edition of Remington's Pharmaceutical Sciences (Maack Publishing Co., Easton, PA).

In other embodiments, the dietary supplement is provided as a powder or liquid suitable for adding by the consumer to a food or beverage. For example, in some embodiments, the dietary supplement can be administered to an individual in the form of a powder, for instance to be used by mixing into a beverage, or by stirring into a semi-solid food such as a pudding, topping, sauce, puree, cooked cereal, or salad dressing, for instance, or by otherwise adding to a food e.g. enclosed in caps of food or beverage container for release immediately before consumption. The dietary supplement may comprise one or more inert ingredients, especially if it is desirable to limit the number of calories added to the diet by the dietary supplement. For example, the dietary supplement of the present invention may also contain optional ingredients including, for example, herbs, vitamins, minerals, enhancers, colorants, sweeteners, flavorants, inert ingredients, and the like.

In some embodiments, the dietary supplements further comprise vitamins and minerals including, but not limited to, calcium phosphate or acetate, tribasic; potassium phosphate, dibasic; magnesium sulfate or oxide; salt (sodium chloride); potassium chloride or acetate; ascorbic acid; ferric orthophosphate; niacinamide; zinc sulfate or oxide; calcium pantothenate; copper gluconate; riboflavin; beta-carotene; pyridoxine hydrochloride; thiamin mononitrate; folic acid; biotin; chromium chloride or picolonate; potassium iodide; sodium selenate; sodium molybdate; phylloquinone; vitamin D3; cyanocobalamin; sodium selenite; copper sulfate; vitamin A; vitamin C; inositol; potassium iodide. Suitable dosages for vitamins and minerals may be obtained, for example, by consulting the U.S. RDA guidelines.

In other embodiments, the present invention provides nutritional supplements (e.g., energy bars or meal replacement bars or beverages) comprising the composition according to the invention. The nutritional supplement may serve as meal or snack replacement and generally provides nutrient calories. Preferably, the nutritional supplements provide carbohydrates, proteins, and fats in balanced amounts. The nutritional supplement can further comprise carbohydrate, simple, medium chain length, or polysaccharides, or a combination thereof. A simple sugar can be chosen for desirable organoleptic properties. Uncooked cornstarch is one example of a complex carbohydrate. If it is desired that it should maintain its high molecular weight structure, it should be included only in food formulations or portions thereof which are not cooked or heat processed since the heat will break down the complex carbohydrate into simple carbohydrates, wherein simple carbohydrates are mono- or disaccharides. The nutritional supplement contains, in one embodiment, combinations of sources of carbohydrate of three levels of chain length (simple, medium and complex; e.g., sucrose, maltodextrins, and uncooked cornstarch).

Sources of protein to be incorporated into the nutritional supplement of the invention can be any suitable protein utilized in nutritional formulations and can include whey protein, whey protein concentrate, whey powder, egg, soy flour, soy milk soy protein, soy protein isolate, caseinate (e.g., sodium caseinate, sodium calcium caseinate, calcium caseinate, potassium caseinate), animal and vegetable protein and hydrolysates or mixtures thereof. When choosing a protein source, the biological value of the protein should be considered first, with the highest biological values being found in caseinate, whey, lactalbumin, egg albumin and whole egg proteins. In a preferred embodiment, the protein is a combination of whey protein concentrate and calcium caseinate. These proteins have high biological value; that is, they have a high proportion of the essential amino acids. See Modern Nutrition in Health and Disease, eighth edition, Lea & Febiger, publishers, 1986, especially Volume 1, pages 30-32. The nutritional supplement can also contain other ingredients, such as one or a combination of other vitamins, minerals, antioxidants, fiber and other dietary supplements (e.g., protein, amino acids, choline, lecithin, omega-3 fatty acids).

Selection of one or several of these ingredients is a matter of formulation, design, consumer preference and end-user. The amounts of these ingredients added to the dietary supplements of this invention are readily known to the skilled artisan. Guidance to such amounts can be provided by the U.S. RDA doses for children and adults. Further vitamins and minerals that can be added include, but are not limited to, calcium phosphate or acetate, tribasic; potassium phosphate, dibasic; magnesium sulfate or oxide; salt (sodium chloride); potassium chloride or acetate; ascorbic acid; ferric orthophosphate; niacinamide; zinc sulfate or oxide; calcium pantothenate; copper gluconate; riboflavin; beta-carotene; pyridoxine hydrochloride; thiamin mononitrate; folic acid; biotin; chromium chloride or picolonate; potassium iodide; sodium selenate; sodium molybdate; phylloquinone; vitamin D3; cyanocobalamin; sodium selenite; copper sulfate; vitamin A; vitamin C; inositol; potassium iodide.

The nutritional supplement can be provided in a variety of forms, and by a variety of production methods. In a preferred embodiment, to manufacture a food bar, the liquid ingredients are cooked; the dry ingredients are added with the liquid ingredients in a mixer and mixed until the dough phase is reached; the dough is put into an extruder, and extruded; the extruded dough is cut into appropriate lengths; and the product is cooled. The bars may contain other nutrients and fillers to enhance taste, in addition to the ingredients specifically listed herein.

It is understood by those of skill in the art that other ingredients can be added to those described herein, for example, fillers, emulsifiers, preservatives, etc. for the processing or manufacture of a nutritional supplement.

Additionally, flavors, coloring agents, spices, nuts and the like may be incorporated into the nutraceutical composition. Flavorings can be in the form of flavored extracts, volatile oils, chocolate flavorings, peanut butter flavoring, cookie crumbs, crisp rice, vanilla or any commercially available flavoring. Examples of useful flavoring include, but are not limited to, pure anise extract, imitation banana extract, imitation cherry extract, chocolate extract, pure lemon extract, pure orange extract, pure peppermint extract, imitation pineapple extract, imitation rum extract, imitation strawberry extract, or pure vanilla extract; or volatile oils, such as balm oil, bay oil, bergamot oil, cedarwood oil, walnut oil, cherry oil, cinnamon oil, clove oil, or peppermint oil; peanut butter, chocolate flavoring, vanilla cookie crumb, butterscotch or toffee. In one embodiment, the dietary supplement contains cocoa or chocolate.

Emulsifiers may be added for stability of the nutraceutical compositions. Examples of suitable emulsifiers include, but are not limited to, lecithin (e.g., from egg or soy), and/or mono- and diglycerides. Other emulsifiers are readily apparent to the skilled artisan and selection of suitable emulsifier(s) will depend, in part, upon the formulation and final product. Preservatives may also be added to the nutritional supplement to extend product shelf life. Preferably, preservatives such as potassium sorbate, sodium sorbate, potassium benzoate, sodium benzoate or calcium disodium EDTA are used.

In addition to the carbohydrates described above, the nutraceutical composition can contain natural or artificial (preferably low calorie) sweeteners, e.g., saccharides, cyclamates, aspartamine, aspartame, acesulfame K, and/or sorbitol. Such artificial sweeteners can be desirable if the nutritional supplement is intended to be consumed by an overweight or obese individual, or an individual with type II diabetes who is prone to hyperglycemia.

Moreover, a multi-vitamin and mineral supplement may be added to the nutraceutical compositions of the present invention to obtain an adequate amount of an essential nutrient, which is missing in some diets. The multi-vitamin and mineral supplement may also be useful for disease prevention and protection against nutritional losses and deficiencies due to lifestyle patterns.

Examples of fortified food are cereal bars, chewing gum and bakery items, such as cakes and cookies.

Beverages encompass non-alcoholic and alcoholic drinks as well as liquid preparation to be added to drinking water and liquid food. Non-alcoholic drinks are for instance soft drinks, sport drinks, fruit juices, such as for example orange juice, apple juice and grapefruit juice; lemonades, teas, near-water drinks and milk based drinks, such as for example yoghurt drinks. Examples of liquid food include soups and dairy products, for instance yoghurt.

The dosage and ratios of the compound according to the invention with the definitions and preferences as given above administered via a nutraceutical composition will, of course, vary depending upon known factors, such as the physiological characteristics of the particular composition and its mode and route of administration; the age, health and weight of the recipient; the nature and extent of the symptoms; the kind of concurrent treatment; the frequency of treatment; and the effect desired which can be determined by the expert in the field with normal trials, or with the usual considerations regarding the formulation of a nutraceutical composition.

In a preferred embodiment, the nutraceutical composition comprises per serving an amount of 0.01 to 1 g, more preferably 0.2 mg to 500 mg of a compound according to the invention with the definitions and preferences as given.

The nutraceutical compositions according to the present invention may be in any galenic form that is suitable for administering to the animal body including the human body, more in particular in any form that is conventional for oral administration, e.g. in solid form, for example as (additives/supplements for) food or feed, food or feed premixes, fortified food or feed, tablets, pills, granules, dragées, capsules, and effervescent formulations such as powders and tablets, or in liquid form, for instance in the form of solutions, emulsions or suspensions, for example as beverages, pastes and oily suspensions. The pastes may be filled into hard or soft shell capsules. Examples for other application forms are forms for transdermal, parenteral, topical or injectable administration. The nutraceutical compositions may be in the form of controlled (delayed) release formulations.

### Description of the figures:

**Figure 1****:** Compound **1** is a novel selective LXRα agonist. **A**, Transcriptional activation of LXRα by T0901317 (T09), 22-R-hydroxycholesterol or compound **1** in a reporter gene assay (see also Table 1). Data are expressed as mean±SD (n=3). **B**, Validation of LXRα specificity. Transcriptional activation of LXRβ by T0901317 (T09) or compound **1** in a reporter gene assay. Compound **1** does not activate the LXRβ subtype (mean±SD, n=3). **C**, Cytotoxicity of compound **1** in macrophages (mean±SD, n=3). Compound **1** does not reduce cellular viability up to 25 µM.
**Figure 2****:** Compound **1** specifically targets diseased, oxysterol-loaden foam cells. **A**, Gene expression in foam cells after siRNA-mediated, single and combined LXRα/β knockdown (mean±SEM, n=4, fold-change vs. DMSO, logarithmised). Left, efficiency of LXRα, LXRβ and LXRα/ β knockdown (kd). Middle, LXRα, LXRβ and LXRα/ β knockdown (kd). Middle, single and combined knockdown influence on gene expression of LXRα, LXRβ and ABCA1 upon T09 treatment. Right, single and combined knockdown influence on gene expression of LXRα, LXRβ and ABCA1 upon compound **1** treatment. * P<0.05, ** P<0.01, *** P<0.001 vs. negative siRNA. **B**, Gene expression in THP-1 macrophages and foam cells after treatment with T0901317 (10 µM) or compound **1** (10 µM). Data are expressed as mean±SEM (n=4). **C**, Western blot analysis of LXRα and APOE content in foam cells and compound **1** treated foam cells. Bar plot displays the results of densitometry analysis (mean±SEM, n=3). * P<0.05, ** P<0.01 vs. foam cell..**D**, Transcriptional activation of LXRα by compound **1** in the presence or absence of 200 nM T09 (see also table **1**). Data are expressed as mean±SD (n=3). **E**, Gene expression in THP-1 macrophages after treatment with different concentrations of T09 or compound **1** in the presence or absence of 1 µM T09. Data are expressed as mean±SEM (n=2-3). * P<0.05, ** P<0.01, *** P<0.001 vs. DMSO; n.s., not significant.
**Figure 3****:** Compound **1** specifically regulates gene expression in foam cells. **A**, Genome-wide gene expression and subsequent gene set enrichment analysis (GSEA) of macrophages and foam cells after treatment with T09 (10 µM) or compound **1** (10 µM). Regulation of lipid-derived Reactome and KEGG pathways is shown. **B**, Validation of gene expression microarray analysis by quantitative PCR. For all tested samples, array observations significantly correlated with qPCR results. **C**, Gene distance matrix of genome-wide gene expression analysis of macrophages and foam cells after treatment with DMSO (0.1 %), T09 (10 µM) or compound **1** (10 µM). Pairwise distances were calculated for comparison of two treatments and cell types. Colored squares show the distance in Euclidean space, ranging from exactly the same profile (black) to completely different (red). In macrophages compound **1** expression is very different to that of T09 (P<0.0001), whereas in foam cells compound 1-induced gene expression is similar to that of T09 (P<0.0001).
**Figure 4****:** Physiological analyses of compound **1** treatment confirm unique compound **1** properties in foam cells. **A**, Cholesterol content in foam cells after treatment for 48 h with DMSO (0.1 %), T09 (10 µM) or compound **1** (10 µM). Data are expressed as mean±SEM (n=5-6). **B**, Cholesterol content in macrophages after treatment for 48 h with DMSO (0.1 %), T0901317 (10 µM) or compound **1** (10 µM). Data are expressed as mean±SEM (n=5-6). **C**, Triglyceride content in foam cells after treatment for 48 h with DMSO (0.1 %), T0901317 (10 µM) or compound **1** (10 µM). **D**, Triglyceride content in macrophages after treatment for 48 h with DMSO (0.1%), T09 (10 µM) or compound **1** (10 µM). Data are expressed as mean±SEM (n=5-6). *** P<0.001 vs. DMSO; n.s., not significant.

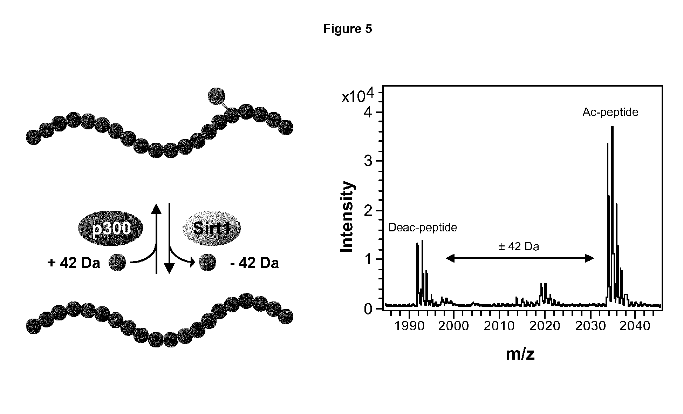

**Figure 5**: Schematic de-/acetylation reaction of peptide substrates by SIRT1/p300. De-/acetylation results in a peptide mass change of 42 Da, which can be observed in mass spectra.

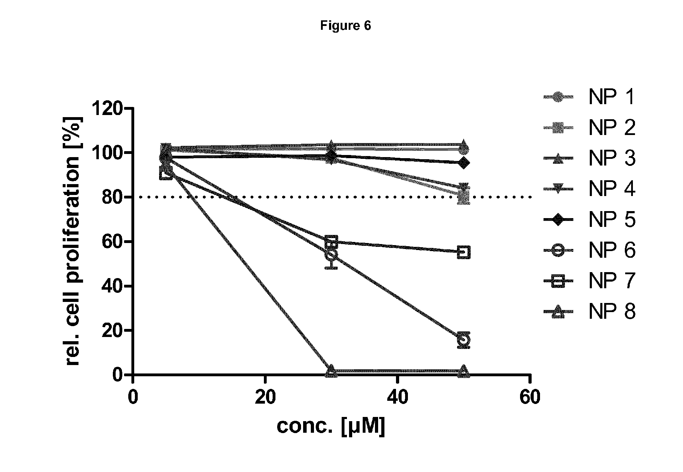

**Figure 6**: HepG2 cells were treated with screening hits (5 µM, 30 µM, 50 µM) for 22 h. Relative cell proliferation below 80% was defined as toxic.
**Figure 7**: a) IC₅₀ curve for NP**1** and SIRT1 generated by MALDI quantification. b) Densitometric analysis of the Western-Blot showing the effect of HepG2 cell treatment with 30 µM NP**1** for 16 h on p53 (Lys382) acetylation state in the nuclear cell fraction (raw data: Figure 15). Basic p53 acetylation was induced by simultaneous treatment with 20 µM Etoposide (Eto).

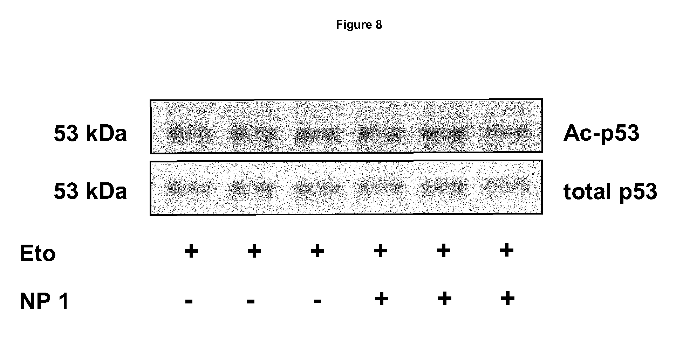

**Figure 8****:** Western-Blot of HepG2 cell treatment with 30 µM NP**1** for 16 h. Basic p53 acetylation was induced by simultaneous treatment with 20 µM Etoposide (Eto). p53 (Lys382) acetylation and total p53 levels were detected in the nuclear cell fraction.

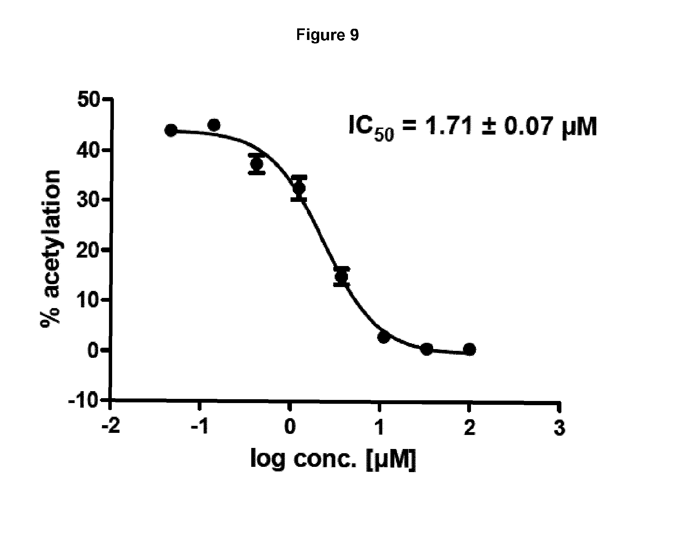

**Figure 9****:** IC₅₀ curve for **NP9** and p300 generated by MALDI quantification
**Figure 10****:** THP-1 cells were treated with 10 µM NP **9**. After 1 hour, LPS was added for 5 hours. TNFα secretion was determined by ELISA.

The following examples are included to demonstrate preferred embodiments of the invention. It should be appreciated by those of skill in the art that the techniques disclosed in the examples which follow represent techniques discovered by the inventor to function well in the practice of the invention, and thus can be considered to constitute preferred modes for its practice. However, those of skill in the art should, in light of the present disclosure, appreciate that many changes can be made in the specific embodiments which are disclosed and still obtain a like or similar result without departing from the spirit and scope of the invention.

Further modifications and alternative embodiments of various aspects of the invention will be apparent to those skilled in the art in view of this description. Accordingly, this description is to be construed as illustrative only and is for the purpose of teaching those skilled in the art the general manner of carrying out the invention. It is to be understood that the forms of the invention shown and described herein are to be taken as examples of embodiments. Elements and materials may be substituted for those illustrated and described herein, parts and processes may be reversed, and certain features of the invention may be utilized independently, all as would be apparent to one skilled in the art after having the benefit of this description of the invention. Changes may be made in the elements described herein without departing from the spirit and scope of the invention as described in the following claims.

### EXAMPLES

Abbreviations used in the present description have the following meanings:
ACN (acetonitrile), CHCA (α-cyano-4-hydroxycinnamic acid), CDCl₃ (deuterated chloroform, Curcumin ((1 *E*,6*E*)-1,7-Bis(4-hydroxy-3-methoxyphenyl)-1,6-heptadiene-3,5-dione), DMSO-*d₆* (deuterated dimethylsulfoxide), DTT (Dithiothreitol), EDTA (Ethylenediaminetetraacetic acid), HEPES (2-(4-(2-Hydroxyethyl)-1-piperazinyl)-ethansulfonic aicd), PEG (poly ethylene glycol), TMS (tetramethylsilane).

### Materials

All chemicals were obtained from commercial suppliers and used without further purification. Compound **1** was synthesized and analysed (using elemental analysis, TLC, mass spectrometry and ¹H and ¹³C NMR) at the Medical University of Vienna by the work-group of Prof. Thomas Erker. Peptides were synthesized by Thermo Fisher Scientific (Ulm) Human recombinant SIRT1 was from Biomol International, Inc. (Plymouth Meeting, PA), Enzo Life Sciences (Lörrach, Germany) and BlueSky BioTech (Worcester, MA). p300, resveratrol and suramin were obtained from Biomol International, Inc. (Plymouth Meeting, PA) / Enzo Life Sciences (Lörrach, Germany). Chemicals were purchased from Sigma Aldrich GmbH (Steinheim, Germany), Carl Roth GmbH + Co. KG (Karlsruhe, Germany) or Merck KGaA (Darmstadt, Germany). DMSO was from AppliChem GmbH (Darmstadt, Germany). Natural product library was from AnalytiCon Discovery GmbH (Potsdam, Germany). α-cyano-4-hydroxycinnamic acid (CHCA), peptide calibration standard II, MALDI target plates and ultraflex II mass spectrometer were from Bruker Daltonik GmbH (Bremen, Germany). Oasis HLB pElution Plates were purchased from Waters (Eschborn, Germany). CellTiter-Glo Luminescent Cell Viability Assay kit was from Promega (Madison, WI). NE-PER Nuclear and Cytoplasmic Extraction Kit and Restore Plus Western Blot Stripping Buffer were purchased from Thermo Scientific (Rockford, IL). NuPAGE Novex 4-12% Bis-Tris Gels were purchased from Invitrogen (Carlsbad, CA), Hybond ECL membrane from GE Healthcare (Freiburg, Germany). The primary antibodies β-actin (C4, sc-47778) and p53 (DO-1, sc-126) and the secondary antibodies goat anti-rabbit IgG-HRP (sc-2004) and goat anti-mouse IgG-HRP (sc-2005) were purchased from Santa Cruz (Heidelberg, Germany). Ac-p53 (Lys382) antibody (#2525S) was from Cell Signaling (Danvers, MA), Western Lightning Plus-ECL solution from Perkin Elmer Inc. (Waltham, MA). Human TNF alpha ELISA Ready-Set-Go! was purchased from eBioscience (San Diego, CA). POLARstar Omega was from BMG LABTECH GmbH (Ortenberg, Germany).

### 1. LXRα agonists

### Example 1-01: LXRα ligand screening

The LXR alpha coactivator kit (Invitrogen) is used and a biologically diverse library of >7,000 compounds for nanomolar binders is screened according to manufacturer's instructions.

### Example 1-02: Cell culture

THP-1, a human acute monocytic leukemia cell line, was obtained from Deutsche Sammlung von Mikroorganismen und Zellkulturen (DSMZ) and cultivated at 37°C (5% CO₂, 95% humidity) in RPMI 1640 (Invitrogen) supplemented with 10% FBS Superior (Biochrom). For generating macrophages differentiation was induced by 10⁻⁸ M Phorbol 12-myristate 13-acetate (PMA) (Sigma-Aldrich) for 48 h. Ligand treatment of macrophages was performed for further 24 h. To induce foam cell formation macrophages were treated for additional 48 h with 100 µg/mL modified human oxidized low density lipoprotein (oxLDL) obtained from Source BioScience (RP-048). Ligand treatment was performed simultaneously with foam cell formation. Cholesterol loading and treatment was controlled with Oil Red O staining (Alfa Aeser). To investigate ligand induced effects on cell proliferation of THP-1 macrophages CellTiter-Glo Luminescent Cell Viability Assay (Promega) was utilized.

For dual luciferase reporter gene assay human embryonic kidney (HEK-293) cells were used that were cultivated in Dulbecco's Modified Eagle Medium (DMEM) with 4,5 g/L glucose (Invitrogen) and 10% FBS Superior at 37 °C (5% CO₂, 95% humidity).

### Example 1-03: Plasmid cloning and dual luciferase reporter gene assay

The ligand binding domain (LBD) of LXRα (amino acids 164 to 447) or LXRβ (amino acids 154 to 461) was cloned into pBIND [Renilla/Amp] vector (Promega). Selection and amplification were performed in *E-coli* cells. Purification of the plasmids was performed with a plasmid purification kit (Qiagen). For transfection HEK-293 cells were plated in 96-well plates (TPP) at a density of 3.5 x 10⁴ cells/well for 24 h. To investigate the ligand binding on LXRα ligand binding domain, 0.04 ng of renilla luciferase coding plasmide pGL4.75 (Promega), 0.04 ng of LXRα-LBD/GAL4-DBD plasmide and 80 ng of firefly luciferase coding plasmide pGL4.31 (Promega) were used in a total volume of 200 µL for each well. In LXRβ binding assay 40 ng of LXRβ-LBD/GAL4-DBD plasmide were applied in each transfection mix. Transfection was carried out for 4 h with Lipofectamine 2000 Transfection Reagent (Invitrogen) in Opti-MEM medium (Invitrogen). Ligand treatment was performed at different concentrations for 24 h. For lysis and detection dual-luciferase reporter gene assay system (Promega) and POLARstar Omega (BMG LABTECH) were used.

### Example 1-04: LXR knockdown

Target specificity of ligand-dependent gene expression effects was investigated in siRNA-mediated LXRα and β-knockdown foam cells with subsequent qPCR or microarray analysis. Therefore, 2 x 10⁵ THP-1 cells/well were differentiated in 24-well plate and induced to foam cells prior to knockdown as described above. Foam cells were transfected using TransIT-TKO transfection reagent (Mirus Bio), for combined knockdown with 15 nM LXRα Silencer Validated siRNA (Ambion, ID 5458) and 15 nM LXRβ Silencer Select Validated siRNA (Ambion, ID s14684) and with 30 nM LXRα or LXRβ for individual knockdowns, respectively. As control we used 30 nM Silencer Select Negative Control #1 (Ambion). Transfection was carried out for 48 h and followed by treatment with 10 µM ligand or vehicle control for 24 h.

### Example 1-05: Real-time polymerase chain reaction

Total RNA isolation and purification was performed by using the RNeasy Mini Kit (QIAGEN) and RNase-Free DNase Set (QIAGEN). cDNA was generated by reverse transcription using the High Capacity cDNA Reverse Transcription Kit (Applied Biosystems). Gene expression was quantified using the SYBR Green PCR Master Mix (Applied Biosystems) and ABI 7900HT Fast Real Time PCR System (Applied Biosystems). In each reaction 0.8 ng/µL cDNA and 0.2 µM primers were used. Gene expression was calculated with the ΔΔCt-method. β-Actin was used for normalisation.

### Example 1-06: Microarray analysis

Microarray analyses were performed according to instructions of Illumina's TotalPrep RNA Amplification Kit followed by hybridisation on HumanHT-12 v3 or HumanHT-12 v4 Expression BeadChips (illumina). Data analysis was performed with GenomeStudio V2011.1 (illumina). Differential expression analysis was performed on background-subtracted data with cubic spline normalization and Benjamini-Hochberg FDR correction. Significant data was considered to have a detection p-value of ≤ 0.01 and differential expression p-value of ≤ 0.05 according to Illumina's t-test error model. Gene expression data were submitted in MIAME-compliant form to the NCBI Gene Expression Omnibus database (GSE39079). Bead array data were validated by quantitative real-time PCR. The Gene Distance Matrix was conducted in Euclidean space using the MeV 4.3 software tool [Saeed A.I. et al. Biotechniques (2003), 34, 374-378]. Gene Set Enrichment Analysis (GSEA) was performed as described elsewhere [Subramanian A. et al. Proc. Natl. Acad. Sci. (2005) 102, 15545-15550] with the following parameters: 1000 gene set permutations, weighted enrichment statistics, minimal gene set size of 5, and signal-to-noise metric. Enrichment of pathways was tested using the Reactome (v3.0) and the KEGG (v3.0) database from the Molecular Signature Database (MSigDB). Heatmaps were carried out with Mayday 2.8 [Battke F. et al. BMC Bioinformatics (2012), 11, 121]. For presentation of treatment effects in the heatmap, the pathway normalized enrichment score (NES) was adjusted with the appropriate FDR as follows: adjustedNES = (1-FDR) x NES.

### Example 1-07: Western blotting

Whole cell extracts were harvested from three biological replicates and denaturized for SDS-PAGE (NuPAGE Novex 4-12% Bis-Tris Gels). Proteins were blotted on nitrocellulose Hybond ECL membrane and incubated with strictly validated published antibodies against LXRα (Abcam, ab 41902), APOE (Abcam ab1906), and as housekeeping protein control β-Actin (Santa Cruz Biotechnology, sc-47778, C4). Secondary antibodies were HRP labeled anti-mouse and anti-rabbit (Santa Cruz Biotechnology). After detection with Western Lightning Plus-ECL solution membranes were stripped with Restore Plus Western Blot Stripping Buffer. Densitometry was performed in ImageQuant TL (GE Healthcare). This tool measures quantitatively the optical density and provides more accurate data than simple eye-observations. The calculation of log (protein of interest/ β-Actin) corrects data for loading imbalances.

### Example 1-08: Cholesterol and triglyceride analyses

THP-1 monocytes were plated out into 24-well plates (Nunc) at a density of 2 x 10⁵ cells/well. After differentiation and treatment cells were lysed with 100 µL lysis buffer (PBS, 0.25 M NaCl, 1% Triton X-100). Total and free cholesterol were determined using the colorimetric enzymatic Amplex Red Cholesterol Assay Kit (Invitrogen). Triglycerides were quantified by Triglyceride Assay Kit (BioVision). For normalisation protein content was determined with Pierce 660 nm Protein Assay Reagent (Thermo Scientific).

### Example 1-09: Statistical analyses

Statistical significance was determined by unpaired two-tailed Student's t-test for single comparisons and one-way ANOVA with Dunnett's post-hoc test for multiple comparisons. Statistical analyses were carried out using GraphPad Prism 5.0. A p-value ≤ 0.05 was defined as statistically significant.

### 2. SIRT1 inhibitor and p300 inhibitor

### Example 2-01 MALDI-TOF MS assay development

### Deacetylation assay

The assay was performed in a 20 µL reaction. 0.5 µM acetylated peptide and 120 µM NAD⁺ in reaction buffer (50 mM Tris-acetate (pH 8), 137 mM NaCl, 2.7 mM KCI, 1 mM MgCl₂, 5 mM DTT, 0.05 % BSA) were mixed with compound or DMSO (VC). Reactions were initiated by adding 10 nM of SIRT1. After incubation for 30 min at 37°C, reactions were stopped by formic acid and nicotinamide (f.c. 1% and 5 mM, respectively). Reactions were performed with histone H4 peptide and 1% DMSO unless stated otherwise.

Screening was performed with p53 peptide and 1.5% DMSO (for pools of five NPs) or 0.3% DMSO (for single NPs).

### Acetylation assay

For IC₅₀ value determination, 250 nM of p300 was mixed with compound or 1% DMSO (VC) and pre-incubated for 30 min at 30°C. Reactions were started by adding 0.5 µM HAT peptide and 120 µM AcCoA and incubated for 60 min at 30°C. After incubation at 30°C for up to 60 min, reactions were stopped by formic acid (f.c. 2%). Reaction buffer consisted of 50 mM HEPES (pH 7.9), 0.1 mM EDTA and 50 µg/mL BSA.

### Calibration curve (CC)

Acetylated and deacetylated peptides were mixed at different ratios in the respective buffer including stop solution and reaction components except the enzyme. Purification, preparation and MS detection were conducted in parallel to reaction samples. CCs were generated for IC₅₀ value determination but not for the screening.

### Peptide purification

Peptides were purified by Oasis HLB µElution Plates using a vacuum manifold [S. Sauer et al. J Chromatogr A (2004), 1049, 9-16]. Plate columns were conditioned by 70% ACN and equilibrated with 0.1% TFA. Samples were loaded, desalted by 0.1 % TFA and H₂O and eluted in 70% ACN.

### Sample preparation

For ultra thin layer (UTL), supernatant of saturated CHCA in ACN / H₂O (2:1) and 0.1% TFA. was mixed with isopropanol (1:3) and was spread on a ground steel target. After solvent evaporated excess of CHCA was removed from target surface with a tissue [D. Fenyo et al. J Vis Exp (2007), 192]. The matrix used for sample preparation contained saturated CHCA in ACN / 0.1% TFA (1:2) or ACN / H₂O (1:2) depending on peptide size [S. L. Cohen et al. Anal Chem (1996), 68, 31-37]. HLB eluted samples and matrix were mixed (1:4) and spotted on the UTL.

### MALDI-TOF MS measurement

Samples were measured automatically (AutoXecute software, Bruker Daltonik GmbH) using an ultraflex II MALDI-TOF mass spectrometer (Bruker Daltonik GmbH) in positive ion reflector mode. Laser power and detector gain were adjusted prior to every automatic run, which were performed in random walk movement.

For assay optimization and IC₅₀ determination, 1000 shots were accumulated in 100 satisfactory shot steps (fuzzy control). At least one peak within a defined mass range had to fulfill the following criteria for the spectra to be accumulated: Absolute (min.) intensity: 800; S/N: 15; peak width: 0.2.

For screening and if fuzzy control measurement was not possible, 1000 shots were accumulated without any criteria.

### Data analysis

A script (Bruker Daltonik GmbH) was employed to analyze peptide peaks in a batch process (flexAnalysis software, Version 3.0, Bruker Daltonik GmbH). Percentage of de-/acetylation were calculated using the sum of the first four monoisotopic peak intensities. CC was generated by plotting experimental vs. calculated peptide acetylation. Measured sample values were interpolated with CC to determine de-/acetylation values (GraphPad Prism). When no CC was created, peak intensity ratios were generated. Verified single NP screening hits had to pass a peak ratio of ≤ 0.1 (deac/ac).

### IC₅₀ value correction

As IC₅₀ values were not gained at initial velocity conditions, values were corrected by a conversion factor according to Wu et al. (Table 5) [G. Wu et al. J Biomol Screen (2003), 8, 694-700].

**Table 5: IC₅₀' value correction (MALDI MS):**

| NP | Obtained IC₅₀' (µM) | Substrate conversion^{[a]} | Conversion factor (IC₅₀' / IC₅₀)^{[b]} | Corrected IC₅₀ (µM) |
|---|---|---|---|---|
| **1** | 13 ± 1 | 43.52% | 1.346 | 9.7 ± 0.7 |
| **2** | 39 ± 13 | 47.80% | 1.399 | 28 ± 9 |
| **3** | 50 ± 8 | 46.33% | 1.380 | 36 ± 6 |
| **4** | 63 ± 5 | 46.33% | 1.380 | 46 ± 4 |
| **5** | 68 ± 4 | 45.34% | 1.368 | 49 ± 3 |
| **9** | 2.35 ± 0.09 | 45.67% | 1.372 | 1.71 ± 0.07 |

| | | | | |
|---|---|---|---|---|
| [a] Substrate conversion: % substrate conversion without compound (DMSO control) [b] Conversion factors were obtained by fitting substrate conversion with conversion factors from Wu et al. using GraphPad Prism. | | | | |

### IC₅₀ value validation

IC₅₀ for **NP 9** was also validated by a radioactivity based assay, and performed by Reaction Biology Corp. (RBC).

### Example 2-02 Cytotoxicity assay

Cytotoxicity was determined by CellTiter-Glo Luminescent Cell Viability Assay kit. HepG2 cells (ATCC) were seeded in a 384-well plate (3.5 x 10³ cells/well). The following day cells were treated with NP concentrations of 5, 30 or 50 µM. After 22 hours of treatment, CellTiter-Glo Reagent was added to each well and luminescence signal was measured (POLARstar Omega). NPs that reduced relative cell proliferation to ≤ 80% were defined as toxic and excluded from further characterization.

### Example 2-03 Western-Blot analysis

HepG2 cells were seeded in 6-well plates (2.5 x 10⁵ cells/well). 48 h later cells were treated with 20 µM Etoposide to induce p53 acetylation and directly afterwards with NP **1** or DMSO (VC) for 16 h. Cells were harvested and separated into cytosolic and nuclear fractions (NE-PER Nuclear and Cytoplasmic Extraction Reagents). Samples were denatured and SDS-PAGE was performed using NuPAGE Novex 4-12% Bis-Tris Gels. Proteins were blotted on nitrocellulose Hybond ECL membrane. Primary antibodies used were β-actin, p53 and acetyl-p53 (Lys382). Secondary antibodies were HRP-conjugated goat anti-rabbit IgG-HRP and goat anti-mouse IgG-HRP. Detection was performed with Western Lightning Plus-ECL solution. Membranes were stripped with Restore Plus Western Blot Stripping Buffer for 15 min.

Densitometry was performed in GelAnalyzer 2010a, which is a software tool to quantitatively measure the optical density. It provides accurate Western-Blot analysis and helps to detect differences which are not visible by the naked eye. Log(p53-Ac/p53-total) was calculated for every sample and statistical analysis was performed.

### Example 2-04 TNFalpha ELISA

THP-1 cells were seeded in a 24-well plate (1.2 x 10⁶ cells/well) in a volume of 0.4 mL. Cells were treated with 10 µM NP **9** or DMSO (VC). After 1 h, 10 ng/mL LPS (Sigma, cat.no. L5293) were added and incubated for another 5 h. Medium was separated from the cells by centrifugation and used for detection of TNFalpha release by a human TNF alpha ELISA Kit [V. M. Nayagam et al. J Biomol Screen (2006), 11, 959-967].

### Example 2-05 Statistical analyses

Data are presented as mean ± SD for MALDI assays and as mean ± SEM for cell based assays. Statistical significance was defined as a P-value < 0.05 (P-values < 0.01 are indicated as **, P-values < 0.001 as ***). For single comparisons the unpaired Student's t-test was used (GraphPad Prism 5.0).

## Claims

1. Compound of the general formula (I) 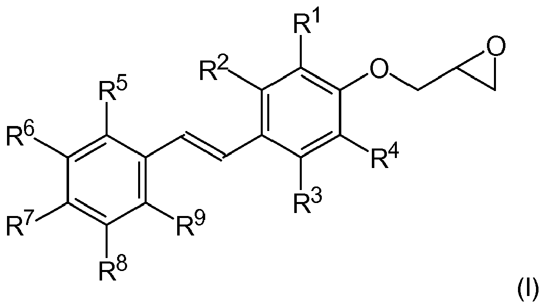 wherein
**R¹ - R⁹** represent independently of each other -H, -F, -Br, -Cl, -I, -CN, **-NO₂, -R³²,** -O**R**³², -S**R**³², -CH₂O**R**³², -CO**R**³², -CO₂**R**³², -SO₂**R**³², -N(**R**³²) **R**³², -CON(**R**³²)**R**³³, -CH₂N(**R**³²) **R**³³, -N(**R**³²)SO₂**R**³³, or - SO₂(**R**³²)N**R**³³;
**R³²** and **R³³** represent independently of each other -H, -CH₃, -C₂H₅, -CH=CH₂, -CH≡CH, -CH₂-CH=CH₂, -C₃H₇, -CH(CH₃)₂, -cyclo-C₃H₅, -C₄H₉, -CH₂-CH(CH₃)₂, -CH(CH₃)-C₂H₅, -C(CH₃)₃, -CH₂F, -CHF₂, -CF₃, -CH₂F₃, or -C₂F₅;
and enantiomers, stereoisomeric forms, mixtures of enantiomers, diastereomers, mixtures of diastereomers, hydrates, solvates, acid salt forms, tautomers, and racemates of the above mentioned compounds and pharmaceutically acceptable salts thereof for use as an agonist of the LXRα protein.

2. Compound according to claim 1, wherein **R**¹- **R**⁹ are -H.

3. Combination of at least one compound according to claim 1 and at least one additional LXRα agonist.

4. Compound according to claim 1 or combination according to claim 3 for use in the treatment or prophylaxis of atherosclerosis and cardiovascular diseases.

5. Compound of the general formula (II) 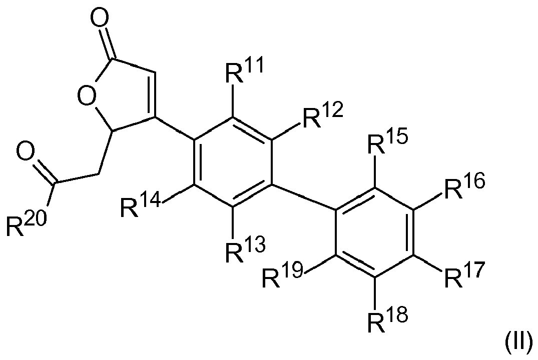 wherein
**R**¹¹ - **R**¹⁹ represent independently of each other -H, -F, -Br, -Cl, -I, -CN, -NO₂, -**R**³², -O**R**³², -S**R**³², -CH₂O**R**³², -CO**R**³², -CO₂**R**³², -SO₂**R**³², -N(**R**³²)R³², -CON(**R**³²)**R**³³, -CH2N(**R**³²)**R**³³, -N(**R**³²)SO₂**R**³³, or - SO₂(**R**³²)N**R**³³;
**R**²⁰ represents -O**R**³² or -N(**R**³²)**R**³³_{;}
and enantiomers, stereoisomeric forms, mixtures of enantiomers, diastereomers, mixtures of diastereomers, hydrates, solvates, acid salt forms, tautomers, and racemates of the above mentioned compounds and pharmaceutically acceptable salts thereof for use as an inhibitor of the SIRT1 enzyme.

6. Compound according to claim 5, wherein **R**¹¹ and **R**¹³ are -OCH₃; **R**¹⁴, **R**¹⁷ and **R**²⁰ are -OH; **R**¹², **R**¹⁵, **R**¹⁶, **R**¹⁸ and **R**¹⁹ are -H.

7. Compound according to claim 5 or 6 for use in the treatment or prophylaxis of neurodegenerative diseases or cancer.

8. Compound according to claim 7, wherein cancer or neurodegenerative disease is selected from Alzheimer's, Huntington's, respiratory diseases, lung cancer and colorectal cancer.

9. Compound of the general formula (III) 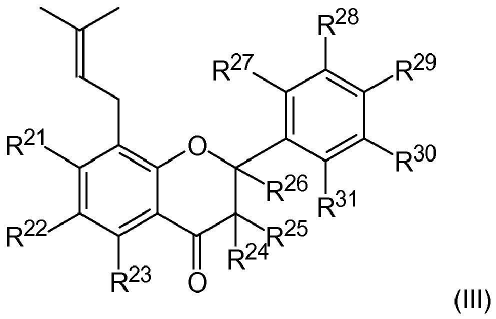 wherein
**R**²¹ - **R**²³ and **R**²⁷- **R**³¹ represent independently of each other -H, -F, -Br, -Cl, -I, -CN, -NO₂, -**R**³², -O**R**³², -S**R**³², -CH₂O**R**³², -CO**R**³², -CO₂**R**³², -SO₂**R**³², -N(**R**³²)**R**³², -CON(**R**³²)**R**³³, -CH₂N(**R**³²)**R**³³, -N(**R**³²)SO₂**R**³³, or -SO₂(**R**³²)N**R**³³;
**R**²⁴ - **R**²⁶ represent independently of each other -H, -OH, or **R**²⁵ and **R**²⁶ form a C=C bond;
and enantiomers, stereoisomeric forms, mixtures of enantiomers, diastereomers, mixtures of diastereomers, hydrates, solvates, acid salt forms, tautomers, and racemates of the above mentioned compounds and pharmaceutically acceptable salts thereof; for use as an inhibitor of the p300 protein.

10. Compound according to claim 9, wherein **R**²², **R**²⁷, **R**²⁸ and **R**³¹ are -H; **R**²¹, **R**²³, **R**²⁴, **R**²⁹ and **R**³⁰ are -OH; **R**²⁵ and **R**²⁶ form a C=C bond.

11. Compound according to claim 9 or 10 for use in the treatment or prophylaxis of cancer, metabolic, inflammatory, neurodegenerative or cardiovascular diseases.

12. Compound according to claim 11, wherein metabolic, inflammatory, cardiovascular, cancer or neurodegenerative disease is selected from type 2 diabetes, atherosclerosis, optic neuritis, ALS, Alzheimer's, Huntington's, Parkinson's, ophthalmic, respiratory diseases, acute leukemia, pancreatic cancer, breast cancer, colorectal cancer, gastric cancer and ovarian cancer.

13. Compound according to claim 1, 2, 5, 6, 9, or 10 for use as pharmaceutically active agent in medicine.

14. Pharmaceutical composition containing at least one compound according to claim 1, 2, 5, 6, 9, or 10 or the combination according to claim 3 together with at least one pharmaceutically acceptable carrier, excipient and/or diluent.

15. Nutraceutical composition containing at least one compound according to claim 1, 2, 5, 6, 9, or 10 or the combination according to claim 3 together with at least one nutraceutical acceptable carrier and/or excipient.
